# EUROPEAN PATENT SPECIFICATION

(11) **EP 3 598 978 B1**
(45) Date of publication and mention of the grant of the patent: **29.05.2024**
(21) Application number: 18185876.2
(22) Date of filing: 26.07.2018
(51) Int. Cl.: A61K 35/28, A61K 31/7105, A61P 21/00

(54) **FIBROADIPOGENIC PROGENITOR-DERIVED EXOSOMES FOR REGENERATION OF DYSTROPHIC MUSCLES**
AUS FIBROADIPOGENEM VORLÄUFER STAMMENDE EXOSOMEN ZUR REGENERIERUNG VON DYSTROPHISCHEN MUSKELN
EXOSOMES DÉRIVÉES DE CELLULES FIBROADIPOGÉNIQUES PROGÉNITRICES POUR LA RÉGÉNÉRATION DES MUSCLES DYSTROPHIQUES

(43) Date of publication of application: 29.01.2020
(73) Proprietor: EXOFIX S.r.l., 00184 Roma (IT)
(72) Inventor: SANDONÀ, Martina, 00052 Cerveteri (RM) (IT); SACCONE, Valentina, 00123 Roma (IT); CONSALVI, Silvia, 00043 Ciampino (RM) (IT)
(74) Representative: Cantaluppi, Stefano

(56) References cited:
- WO-A2-2017/163132
- US-A1- 2017 290 860
- "Advanced Methods to Study the Cross Talk Between Fibro-Adipogenic Progenitors and Muscle Stem Cells." In: TUCCIARONE LUCA ET AL: "Duchenne Muscula Dystrophy, Methods and Protocols", 25 October 2017 (2017-10-25), METHODS IN MOLECULAR BIOLOGY (CLIFTON, N.J.) 2018, VOL. 1687, PAGE(S) 231 - 256, XP9509583, ISSN: 1940-6029 ISBN: 978-1-4939-7373-6 pages 231-256, DOI: 10.1007/978-1-4939-7374-3_17, * 3.1.4 MuSC Culture with FAP exosomes *
- LESLEY CHENG ET AL: "Exosomes provide a protective and enriched source of miRNA for biomarker profiling compared to intracellular and cell-free blood", JOURNAL OF EXTRACELLULAR VESICLES, vol. 3, no. 23743, 26 March 2014 (2014-03-26), pages 1-14, XP055239395, DOI: 10.3402/jev.v3.23743
- CHIARA MOZZETTA ET AL: "Fibroadipogenic progenitors mediate the ability of HDAC inhibitors to promote regeneration in dystrophic muscles of young, but not old Mdx mice : Stage-specific effect of HDACi in mdx mice", EMBO MOLECULAR MEDICINE, vol. 5, no. 4, 18 March 2013 (2013-03-18), pages 626-639, XP055526807, Weinheim ISSN: 1757-4676, DOI: 10.1002/emmm.201202096
- SACCONE VALENTINA ET AL: "HDAC-regulated myomiRs control BAF60 variant exchange and direct the functional phenotype of fibro-adipogenic progenitors in dystrophic muscles", GENES AND DEVELOPMENT, COLD SPRING HARBOR LABORATORY PRESS, PLAINVIEW, NY, US , vol. 28, no. 8 15 April 2014 (2014-04-15), pages 841-857, XP009509586, ISSN: 0890-9369, DOI: 10.1101/GAD.234468.113 Retrieved from the Internet: URL:http://genesdev.cshlp.org/cgi/doi/10.1 101/gad.234468.113 [retrieved on 2014-03-28]
- GIORDANI L ET AL: "Muscle-specific microRNAs as biomarkers of Duchenne Muscular Dystrophy progression and response to therapies", RARE DISEASES, TAYLOR & FRANCIS INC, US , vol. 2, no. 1 1 January 2014 (2014-01-01), pages e974969-1, XP009509585, ISSN: 2167-5511, DOI: 10.4161/21675511.2014.974969 Retrieved from the Internet: URL:http://www.tandfonline.com/doi/full/10 .4161/21675511.2014.974969 [retrieved on 2014-12-01]
- SHALOME BASSETT ET AL: "The Role of Dietary Histone Deacetylases (HDACs) Inhibitors in Health and Disease", NUTRIENTS, vol. 6, no. 10, 15 October 2014 (2014-10-15), pages 4273-4301, XP055526873, DOI: 10.3390/nu6104273

## Description

The present invention relates to the fields of pharmaceuticals and biotechnology.

In particular, the present invention refers to the treatment of muscular dystrophies, such as Duchenne Muscular Dystrophy.

### Background of the invention

Duchenne muscular dystrophy (DMD) is a severe type of muscular dystrophy. The symptom of muscle weakness usually begins around the age of three-four in boys and worsens quickly. Typically muscle loss occurs first in the upper legs and pelvis followed by those of the upper arms. This can result in trouble standing up. Most are unable to walk by the age of 12.

This genetic disease is characterised by skeletal muscle degeneration and progressive muscle wasting, which is caused by loss-of-function mutations in the dystrophin gene. Dystrophin has critical roles in myofiber stability and integrity by connecting the actin cytoskeleton to the extracellular matrix. Absence of dystrophin leads to progressive defects including perturbation of the calcium homeostasis, activation of proteases and pro-inflammatory cytokines, and mitochondrial dysfunction resulting in continual influx of inflammation, fibrosis, repeated cycles of necrosis and altered regeneration, with impaired vascular adaptation. The myofibres become more susceptible to contraction-induced injury, which results in premature death, muscle wasting and fatty tissue replacement. Accumulating evidence also indicates that muscle stem (satellite) cells (Mauro, 1961) (therein indicated as MuSCs) are defective in dystrophic muscles, which leads to impaired muscle regeneration (Dumont and Rudnicki, 2016) (Mauro, 1961).

No cure for muscular dystrophy is known and an ongoing medical need has been recognized by regulatory authorities.

Treatment is generally aimed at controlling the onset of symptoms to maximize the quality of life.

Therapies targeting MuSCs or the environment around MuSCs hold great potential and could have long-term efficiency owing to the high self-renewal ability of these cells.

Emerging evidence indicates that reciprocal interactions between distinct cellular components of the regeneration machinery generate either productive or hostile environment for regeneration of dystrophic muscles (Moyer and Wagner, 2011; Tidball, 2011; Farup et al., 2015).

While MuSCs are the direct effectors of muscle repair, a variety of other cell types contribute to muscle regeneration by spatially and temporally coordinating the activity of MuSCs (Dumont et al., 2015a). These cells comprise components of the inflammatory infiltrate, including macrophages and eosinophils (Tidball et al., 2014; Kharraz et al., 2013), and a recently identified population of muscle-derived interstitial cells referred to as fibro-adipogenic progenitors (FAPs) (Joe et al., 2010; Uezumi et al., 2010). The reciprocal interplay between these cell types generates a network of signals that coordinate sequential stages of muscle repair and provides the "functional niche" of MuSCs (Bentzinger et al., 2013).

Disruption of this network compromises the integrity of MuSC niche and has been associated with the progression of many chronic muscular disorders (i.e. muscular dystrophies) and age-related decline in muscle mass and repair (Blau et al., 2015, Uezumi et al., 2011).

For instance in Duchenne Muscular Dystrophy (DMD) the gradual exhaustion of the compensatory regeneration coincides with changes in the muscle tissue composition, leading to the progressive formation of fibrotic and adipose tissue in place of contractile fibers (Hoffman and Dressman, 2001).

The association of such "restriction point" in the natural history of DMD with alterations of functional interactions between cellular components of the MuSC niche has inspired pharmacological strategies aimed at targeting relevant cellular networks, as an alternative or complementary approach to strategies toward restoring dystrophin expression (Ruegg, 2013).

In view of the above, there is still the need of a therapeutic tool for treating muscular dystrophies, such as DMD, in particular to prevent their progression.

More in particular, there is the need of a therapeutic instrument able to reactivate regeneration of muscle cells and avoid or slow the progressive formation of fibrotic and adipose tissue in place of contractile fibers.

Defining the identity of extracellular mediators that coordinate the activities of the cellular components of the muscle stem cell niche is a major challenge in regenerative medicine.

Indeed, this would be highly desirable in order to develop selective interventions toward promoting compensatory regeneration of diseased muscles, while eliminating the most unfavorable outcome of disease progression - the maladaptive repair by deposition of fibrotic and adipose tissue that typically compromises contractile activity and regenerative potential of muscles.

While a variety of soluble factors, including inflammatory cytokines and growth factors, have been implicated as mediators of reciprocal functional interactions between the major cellular components of the muscle stem cell niche, EVs (e.g. exosomes) are emerging as powerful tool to transfer biochemical and genomic information from one cell type to another (Lee et al., 2012; Braicu et al., 2015; Iraci et al., 2016). Indeed, by virtue of their content in proteins and RNA, exosomes enable the cross-activation of biological process between neighbor cells that accounts for their functional interactions. As such, the qualitative and quantitative control of proteins and RNA content appears as a major determinant of exosome biological activity. Likewise, the direction of cell-to-cell transmission of exosomes influences the final biological outcome of exosome activity.

For example, previous work has shown that MuSC-derived exosomes containing miR-206 are required to eliminate fibrosis during muscle hypertrophy (Fry et al., 2017). Moreover, myofiber-derived exosomes have been reported to promote skeletal myogenesis (Choi et al 2016).

Recent studies have revealed the "pivotal" function of FAPs within the regenerative network composed of cellular contributors of muscle regeneration.

Upon myofiber damage, FAP accumulation is preceded by the appearance of the inflammatory infiltrate and is followed by MuSC activation (Lemos et al., 2015; Heredia et al., 2013). This temporal pattern suggests a key role for FAPs in converting inflammatory cues into signals that regulate MuSC activity, and implies reciprocal communications between these cell types, through the exchange of soluble mediators, which are largely unknown.

Our previous studies have revealed that FAPs are key targets of histone deacetylase inhibitors (HDACi) - a pharmacological intervention that counters DMD progression by promoting compensatory regeneration, while inhibiting fibro-adipogenic degeneration both in pre-clinical studies (Minetti et al., 2006; Consalvi et al., 2011; Mozzetta et al., 2013; Consalvi et al., 2013; Saccone et al., 2014;) and clinical trials (Bettica et al. 2016).

While these data indicate the pharmacological potential of HDACi to restore the regenerative environment in dystrophic muscles, by recovering physiological functional interactions between FAPs and other cellular components, the released signals that mediate HDACi ability to restore physiological interactions between FAPs and other cellular components of DMD muscles are still unknown.

However, it is well known that the administration of HDAC inhibitors often leads to collateral effects, toxicity and off-targets activities.

Also, pharmacological interactions of HDAC inhibitors with other drugs used in the treatment of muscular dystrophies, such as for example steroids, are likely.

Therefore, there is still the need of an alternative to the administration of HDAC inhibitors for the treatment of muscular dystrophies.

It has now been found that HDAC inhibitors are able to modulate the content of extracellular vesicles (EVs) that mediate FAP's ability to promote MuSCs activation and differentiation, and to inhibit muscle fibrosis, in DMD muscles. More in particular, it has been found that such exosomes are able to promote MuSCs activation and expansion *ex vivo,* and to stimulate regeneration of dystrophic muscles *in vivo.* Also, it has been found that such exosomes are able to reduce muscle fibrosis and inflammation.

Therefore, they can be advantageously used for selective therapeutic interventions in muscular diseases.

An additional advantage provided by the use of exosomes is that they can be used for local treatments - e.g. targeting specific group of muscles, by topical injection. This avoids the side effects caused by systemic administration of HDACi.

Some patent literature describes the treatment of muscular dystrophies by administering or modulating the expression of some miRNAs, see for example WO2015161255, US20140121171, WO2017181014, WO2017180976, WO2014169126, EP2435583.

It is also known in the patent literature the administration of exosomes of various origins for the treatment of diseases. For example, WO2016054591 discloses the administration of exosomes derived from cardiosphere-derived cells for the treatment of heart failure; WO2015038075 discloses the use of exosomes deriving from muscular stem cells to restore homeostasis; WO2017199250 discloses the use of exosomes deriving from mesenchimal stem cells for the treatment of muscle-associated diseases; WO2014041087 discloses several types of membrane vesicles secreted by muscle cells and their therapeutic uses for example in the treatment of muscular diseases.

However, the use as a therapeutic tool of exosomes modulated in their content by a specific pharmacological intervention was not known in the prior art, in particular for the treatment of muscular diseases.

It has been found that the therapeutic use of such exosomes, obtained in specific conditions and from specific cells, allows to promote specific regeneration of dystrophic muscles.

The use of exosomes derived from progenitor cells has the great advantage of delivering the benefits of stem cell regeneration, without resorting to mechanisms involving administration or transplant of the cell themselves.

### Summary of the invention

It is an object of the invention an isolated extracellular vesicle, preferably an exosome, obtained from fibro-adipogenic progenitors (FAPs) previously exposed to histone deacetylase (HDAC) inhibitors, wherein said histone deacetylase inhibitor is selected from the group consisting of Trichostatin A,MS-275, suberanilohydroxamic acid (SAHA) and Givinostat, for use in the treatment of a muscular dystrophy.

In particular, said vesicle is able to promote regeneration of dystrophic muscles, more in particular regeneration of new muscular fibers.

Said fibro-adipogenic progenitors (FAPs) are previously exposed to histone deacetylase (HDAC) inhibitors wherein said histone deacetylase inhibitor is selected from the group consisting of Trichostatin A,MS-275, suberanilohydroxamic acid (SAHA) and Givinostat by their treatment *in vitro* with said HDAC inhibitor.

Said fibro-adipogenic progenitors (FAPs) are previously exposed histone deacetylase (HDAC) inhibitors wherein said histone deacetylase inhibitor is selected from the group consisting of Trichostatin A,MS-275, suberanilohydroxamic acid (SAHA) and Givinostat by treatment of a dystrophic subject, animal or human, with said HDAC inhibitor and subsequent isolation of said FAPs from the muscles of said subject.

In a preferred embodiment, said fibro-adipogenic progenitors originate from the dystrophic muscles of a subject.

In a preferred embodiment, said exosome comprises at least one microRNA selected from the group consisting of: miR-206-3p, miR-542-5p, miR-449a-5p, miR-342-3p, miR-320-3p, miR-192-5p, miR-423-5p, miR-376a-3p, miR-145a-5p, miR-224-5p, miR-30a-5p, miR-494-3p, miR-29a-3p and miR-7b-5p.

It is also an object of the invention a composition comprising a plurality of said exosomes.

Another object of the invention is said vesicle for use in the treatment of a muscular dystrophy, more in particular in the treatment of Duchenne Muscular Dystrophy (DMD).

Compositions, vectors and formulations for the administration of said exosome are also within the scope of the invention.

In particular, a pharmaceutical composition containing at least one of said extracellular vesicle or exosome, typically in an amount such as to produce a significant therapeutic effect, together with pharmaceutically acceptable vehicles and/or excipients is also an object of the invention.

Said pharmaceutical composition for use in the treatment of a muscular dystrophy, in particular in the treatment of Duchenne Muscular Dystrophy, is a further object of the invention.

### Description of the invention

### Definitions

Within the meaning of the present invention, for muscular dystrophy it is intended a muscle disease that results in increasing weakening and breakdown of skeletal muscles over time. In particular, it is intended any disease sharing specific pathogenic events that are targeted by HDACi. It includes DMD and alpha sarcoglycanopathies (e.g. limb-girdle muscular dystrophy (LGMD 2D)) (Minetti et al. 2006; Bettica et al. 2016; Pambianco et al. 2016), and also dystrophies in which genetic deficiency of components of the Dystroglycan-associated complex leads to persistent cycles of degeneration-regeneration post-contraction, thereby causing an activation of FAPs with a pathologic activity that is countered by HDACi.

Within the meaning of the present invention, for Duchenne Muscular Dystrophy a severe type of muscular dystrophy is intended, that is characterized by a mutation of the dystrophin gene at locus Xp21, located on the short arm of the X chromosome.

Within the meaning of the present invention, by "dystrophic muscle", a muscle of a subject affected by a muscular dystrophy is intended.

Within the meaning of the present invention, for "dystrophic subject" it is intended a subject, preferably a boy, affected by a muscular dystrophy.

Within the meaning of the present invention, for "extracellular vesicle" it is intended a population of endogenous, lipid-bound, nanoparticles, which transfer protein and nucleic acids between different cell types. Extracellular vesicles are heterogenous for their size and they include exosomes, microvesicles and apoptotic bodies.

Within the meaning of the present invention, for "exosome" it is intended a cell-derived vesicle that is present in all eukaryotic fluids, including blood, urine, maternal milk and cultured medium of cell cultures. Exosomes are smaller Extracellular Vesicles (EVs), with a range of size typically between 30 and 150 nm, a characteristic buoyant density typically approximately of 1.10 to 1.21 g/ml in sucrose and aside their lipids such as ceramide, cholesterol and sphingolipids, their composition typically comprises proteins like Rab GTPases, heat shock protein 70, annexins, TSG101, Alix and tetraspanins (CD63, CD9, CD81 and CD82) (Théry et al., 2002).

Within the meaning of the present invention, by "fibro-adipogenic progenitors (FAPs)" a mesenchymal population of pluripotent muscle-derived interstitial cells is intended. These cells can be isolated by fluorescence activated cell sorting (FACS) as CD45-/ CD31- (lineage-negative lin-)/ Sca1+/ a7integrin- or as lin-/ PDGFRa+/SM/C-2.6- cells.

Within the meaning of the present invention, by "muscle stem cells (MuSC)" a cell population is intended that resides between the basal lamina and sarcolemma of myofibers and that is characterized by the expression of Pax3 and its paralogue Pax7.

### Figures

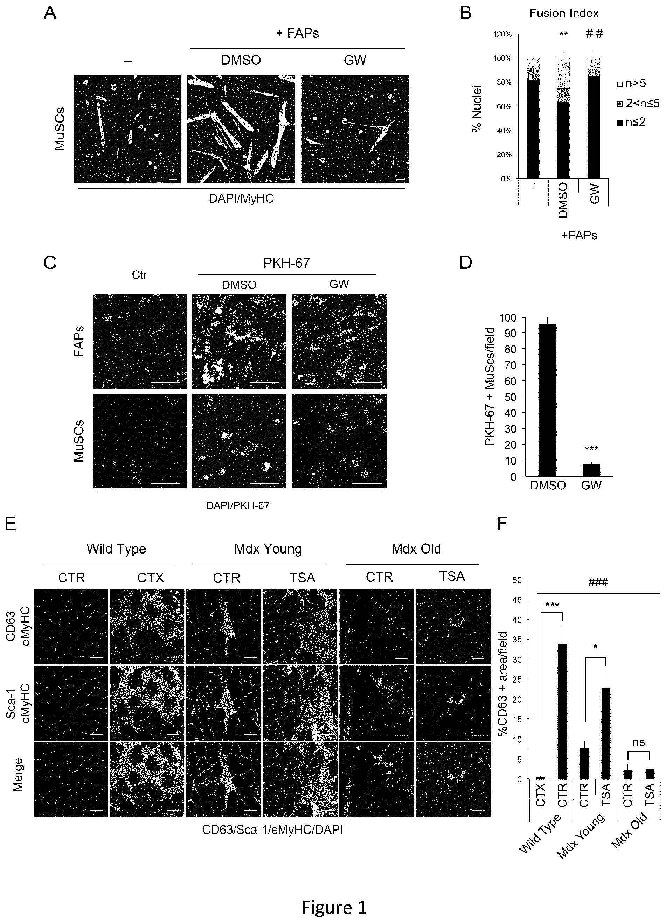

Figure 1: Extracellular vesicles from FAPs mediate the functional crosstalk with MuSCs and correlate with muscle regeneration. A) Representative images showing the myogenic differentiation of MuSCs assessed by immunostaining for MyHC (white). Nuclei were counterstained with DAPI (grey). MuSCs were cultured alone (-) or in transwell co-culture with FAPs treated with DMSO or GW4869 (GW). Scale bar = 50 µm. B) Graph showing the fusion index of MuSCs in the conditions described in A. n<2 indicates the percentage of nuclei that were MyHC⁻ or MyHC⁺ in mononucleated myotubes. 2 <n< 5 indicates the % of nuclei that were MyHC⁺ inside myotubes containing between 2 and 5 nuclei. n>5 indicates the % of nuclei that were MyHC⁺ inside myotubes containing more than 5 nuclei. Star (*) indicates statistical analysis by t-test relative to MuSCs cultured alone (-). ** p < 0.01. Hash (#) indicates statistical analysis by t-test relative to MuSCs in co-cultured with FAPs not treated (DMSO). ## p<0.01. C) Representative images of PKH-67 (white) and DAPI (grey) staining in a transwell co-culture experiment showing EVs/exosomes transfer from FAPs to MuSCs. FAPs have been stained with PKH-67 or not (Ctr) and treated with DMSO or GW4869 to inhibit EVs biogenesis. Scale bar = 25 µm. D) Graph showing the percentage of PKH-67 positive MuSCs after the co-culture with FAPs treated with DMSO or GW4869 (GW) as described in C. Star (*) indicates statistical analysis by t-test. *** p < 0.001. E) Representative images of immunofluorescence for CD63 (dark grey), Sca-1 (white) and eMyHC (light grey) in muscle transversal sections of tibialis anterior from wild type mice (control -CTR- and cardiotoxin injured -CTX-), mdx young and old mice (control -CTR- and TSA treated). Scale bar = 50 µm. F) Graph relative to the quantification of CD63 positive signal showed in E. Star (*) indicates statistical analysis by t-test. *p < 0.05; *** p < 0.001. Hash (#) indicates statistical analysis by anova. ### p < 0.001. ns = not significant.

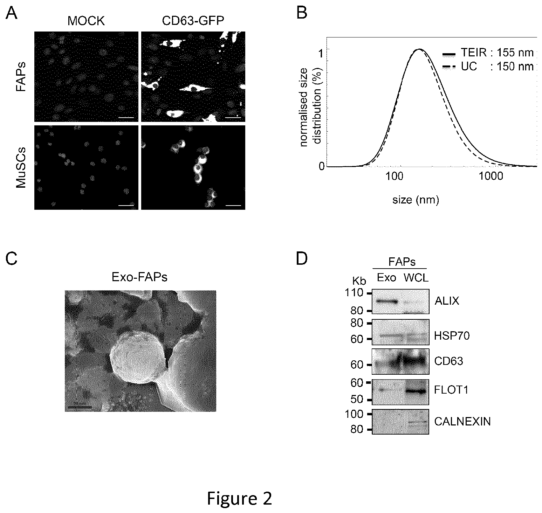

Figure 2: FAPs release Extracellular vesicles with physical features of exosomes that are transferred to MuSCs. A) Representative images of GFP detection (white) in MuSCs and FAPs co-cultured in transwell. Nuclei were counterstained with DAPI (grey). FAPs were previously transfected with mock (control GFP-plasmid) or GFP-CD63 plasmid. Scale bar = 25 µm. B) Dynamic light scattering (DLS) analysis showing the size of FAPs-derived extracellular vesicles (EVs) isolated by total extracellular vesicles isolation reagent (TEIR) or ultracentrifugation (UC). C) Scanning electron microscopy of an exosome purified from FAPs cell culture serum free media using TEIR. D) Exosomes characterization by Western blot analysis for Alix, Hsp70, CD63, Flotilin1 (Flot1) and Calnexin antibodies in exosomes purified from FAPs cell culture serum free media (Exo) and in the cell lysate (WCL).

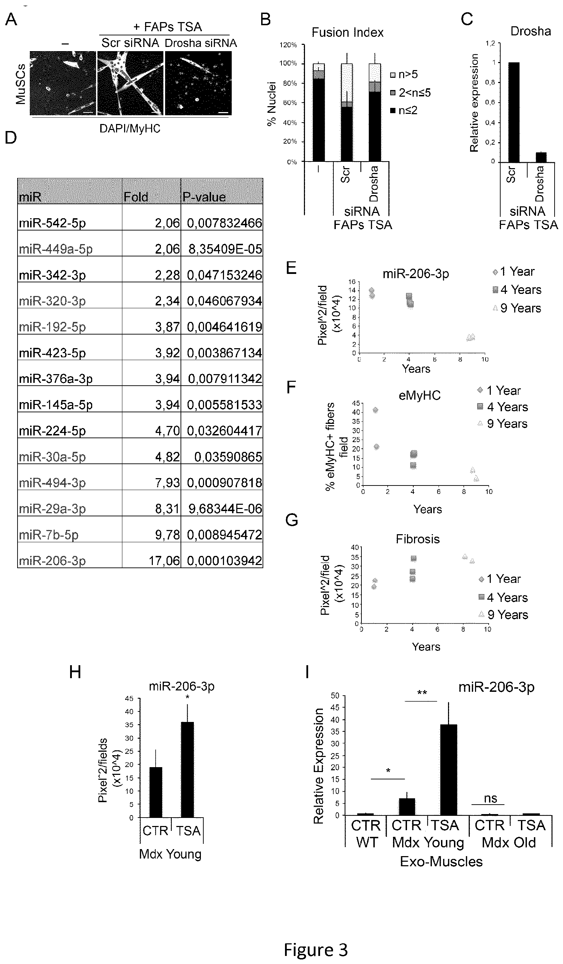

Figure 3: FAPs derived exosomes isolated from HDACi treated mdx mice contain miR-206-3p that correlate with muscle regeneration and DMD progression. A) Representative images of myogenic differentiation of MuSCs assessed by immunostaining for MyHC (white). Nuclei were counterstained with DAPI (grey). MuSCs were cultured alone (-) or in transwell co-culture with FAPs isolated from TSA treated mdx, treated with scramble (Scr siRNA) or Drosha siRNA (Drosha siRNA). Scale bar = 50 µm. B) Graph showing the fusion index of MuSCs in the conditions described in A. n<2 indicates the percentage of nuclei that were MyHC⁻ or MyHC⁺ in mononucleated myotubes. 2 <n< 5 indicates the % of nuclei that were MyHC⁺ inside myotubes containing between 2 and 5 nuclei. n>5 indicates the % of nuclei that were MyHC⁺ inside myotubes containing more than 5 nuclei. C) Graph showing the relative expression of Drosha in FAPs from mdx mice treated with TSA after Drosha down-regulation by siRNA. D) Table representing a manually assembled list of microRNAs revealed by microarray analysis and statistically induced by TSA in FAPs-derived exosomes. E) Graph representing the distribution of miR-206-3p positive area quantification measured as pixel^2/field relative to DMD patients' age: DMD patients of 1 year old n=2; DMD patients of 4 years old n=3, DMD patients of 9 years old n=2. F) Graph showing the distribution of eMyHC positive fibers percentage of DMD patients at different ages: DMD patients of 1years old n=2; DMD patients of 4 years old n=3, DMD patients of 9 years old n=2 G) Graph showing the distribution of fibrosis area quantification, measured as pixel^2/field, relative to DMD patients' age: DMD patients of 2 years old n=2; DMD patients of 4 years old n=3, DMD patients of 9 years old n=2. H) Graph showing the quantifications of miR-206-3p positive area measured as pixel^2/field relative to sections of mdx mice (1.5 months old) treated or not with TSA (n=3). Statistical significance tested by t-test. Star (*) indicates statistical analysis by t-test. * p < 0.05. I) Graph representing the miR-206 relative expression in exosomes isolated from muscle interstitium of tibialis anterior from wild type mice (CTR), mdx young and old mice (control -CTR- and TSA treated). Star (*) indicates statistical analysis by t-test. *p < 0.05; ** p < 0.01; ns = not significant.

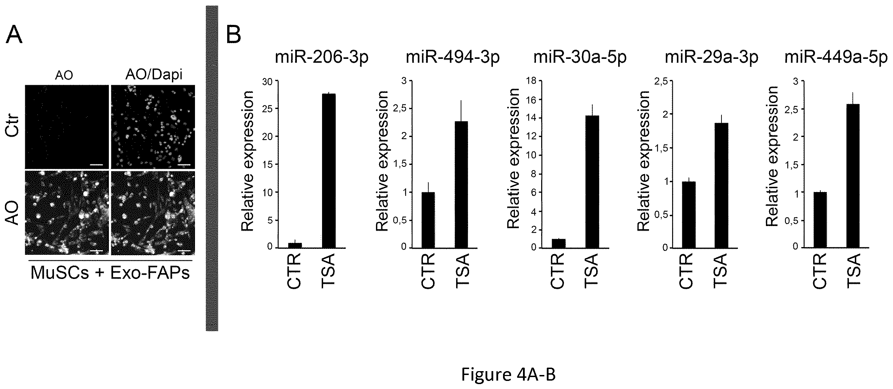

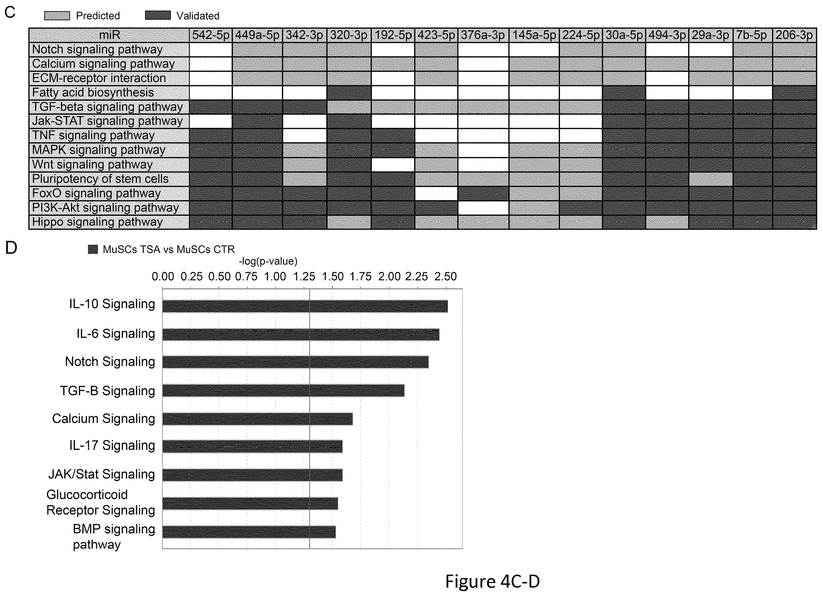

Figure 4: micro-RNAs are vehiculated in exosomes from FAPs to MuSCs. A) MuSCs uptake of RNA from FAPs-derived exosomes (Exo-FAPs). Exosomes harvested from FAPs donor cells were stained with Acridine Orange fluorescent dye (AO- white signal) or not (Ctr) and incubated with MuSCs. Nuclei were counterstained with DAPI (grey). Scale bar = 50 µm. B) Graphs showing the relative expression of microRNAs resulted up-regulated in Exosomes from FAPs by TSA treatment. C) Table representing the Pathway Analysis of TSA upregulated miRNAs induced in FAPs-derived exosomes based on their predicted (shown in light grey) or already validated (shown in dark grey) targets. D) Ingenuity Pathway Analysis of canonical pathways found significatively modulated in MuSCs TSA versus MuSCs CTR.

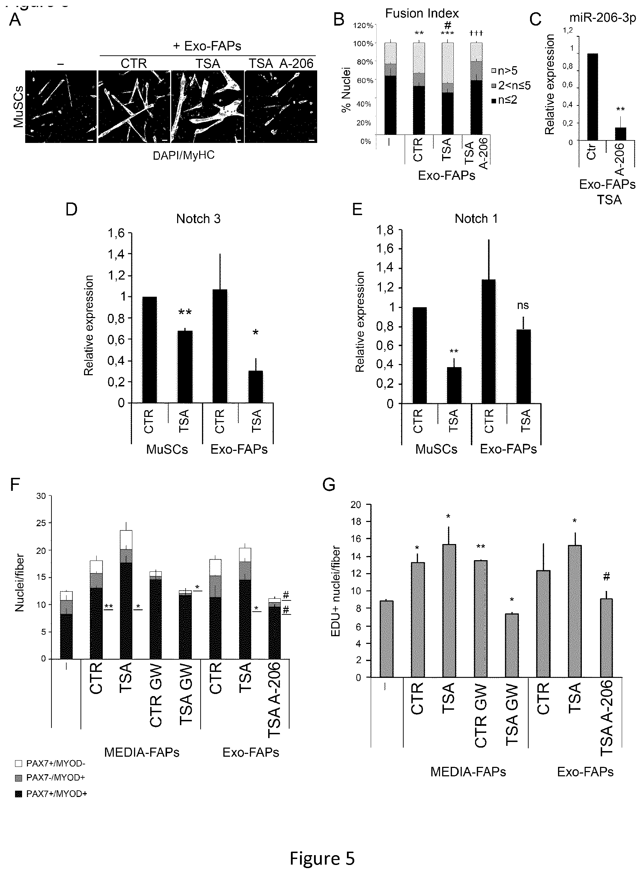

Figure 5: Exosomes from FAPs *in vivo* exposed to TSA promote MuSCs differentiation, expansion and asymmetric division through miR-206-3p. A) Representative images of myogenic differentiation of MuSCs assessed by immunostaining for MyHC (white). Nuclei were counterstained with DAPI (grey). MuSCs were cultured alone (-) or with exosomes (Exo FAPs) isolated from FAPs *in vivo* exposed or not to TSA (CTR and TSA) and subsequently treated with antagomiR-206 (TSA A-206). Scale bar = 50 µm. B) Graph showing the fusion index of MuSCs in the condition described in A. n<2 indicates the percentage of nuclei that were MyHC⁻ or MyHC⁺ in mononucleated myotubes. 2 <n< 5 indicates the % of nuclei that were MyHC⁺ inside myotubes containing between 2 and 5 nuclei. n>5 indicates the % of nuclei that were MyHC⁺ inside myotubes containing more than 5 nuclei. Star (*) indicates statistical analysis by t-test relative to MuSCs cultured alone (-). Star (*) means significance compared to MuSCs alone, **p < 0.01, ***p>0,001; hash (#) means significance compared to Exo-FAPs CTR, #p<0,05; cross (†) indicates significance compared to Exo-FAPs TSA, †††p<0,001. C) Graph showing the relative amount of miR-206-3p in Exosomes isolated from FAPs of mdx mice treated with TSA (Exo-FAPs TSA): control (Ctr) or transfected with antagomiR-206 (A-206). Star (*) indicates statistical analysis by t-test; **p < 0.01. D) Graph relative to Notch3 expression in MuSCs isolated from mdx vehicle treated (CTR) and from TSA daily treated mdx mice and to exosomes from FAPs (Exo-FAPs) isolated from vehicle (CTR) and TSA daily treated mdx animals. Star (*) indicates statistical analysis by t-test relative to mdx vehicle treated mdx mice (CTR); *p<0,05; **p < 0.01. E) Graph relative to Notch1 expression in MuSCs isolated from mdx vehicle treated (CTR) and from TSA daily treated mdx mice and to exosomes from FAPs (Exo-FAPs) isolated from vehicle (CTR) and TSA daily treated mdx animals. Star (*) indicates statistical analysis by t-test relative to mdx vehicle treated mdx mice (CTR); **p < 0.01. ns = not significant. F) Graph showing the average of nuclei positive for Pax7 (white), MyoD (Light grey) or both (dark grey) for single fiber cultured for 24 hours alone (-) or with conditioned media of FAPs that were previously exposed or not to TSA and GW4869 (MEDIA-FAPs) or exosomes (Exo-FAPs) collected from FAPs exposed or not to TSA *in vivo* (CTR and TSA) and transfected with antagomiR-206 (TSA A-206). Star (*) indicates statistical analysis by t-test relative to myofibers cultured alone (-). * p < 0.05, ** p < 0.01. Hash (#) indicates statistical analysis t-test relative to Exo-FAPs TSA. # p < 0.05. G) Graph showing the average of EDU positive nuclei for single fiber in the experimental points indicated in F. Statistical analysis is shown as described in F.

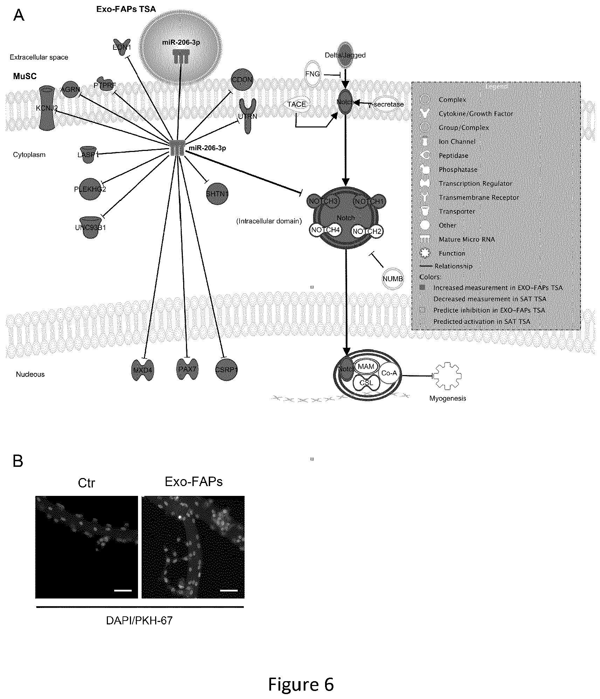

Figure 6: Exosomal miR-206-3p potential targets in MuSCs. A) Cartoon showing the putative activity of miR-206-3p released in exosomes from FAPs exposed to TSA on MuSCs targets. The Cartoon was generated by Ingenuity Pathway Analysis intersecting RNAseq data from MuSCs-TSA with Exo-FAPs TSA microarray. Notch3 signaling modulation is highlighted on the right. B) Representative images showing myofibers cultured alone (Ctr) or with exosomes isolated from FAPs (Exo-FAPs) and stained with PKH-67 (light grey). Scale bar = 50 µm.

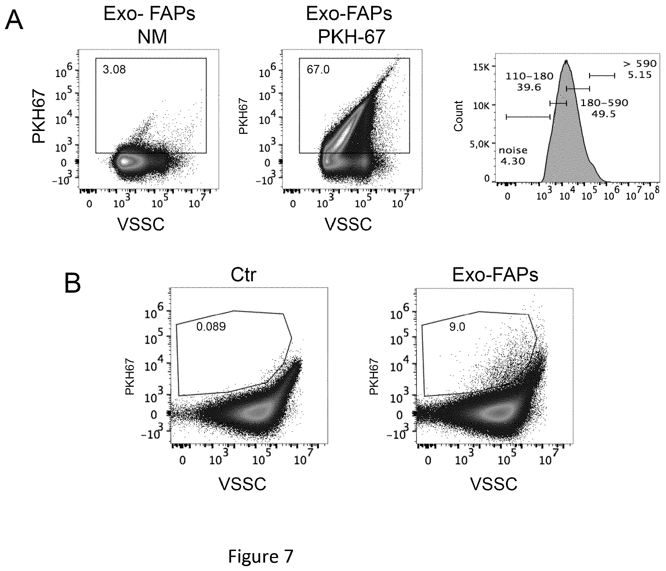

Figure 7: FAPs exosomes injection in mdx TA muscle. A) Flow cytometry analysis of exosomes isolated from FAPs and stained or not with PKH-67 lipidic dye. The left panel represents sample without PKH-67 staining (Exo-FAPs NM); middle panel shows sample stained with PKH67 dye (Exo-FAPs PKH-67); right panel shows the size of the PKH-67 positive Exosomes. B) Flow cytometry analysis of digested muscles previously injected with vehicle (Ctr) or with PKH67-labeled exosomes isolated from FAPs (Exo-FAPs). The left panel shows muscle injected with PBS. Right panel shows muscle injected with PKH-67 labeled exosomes.

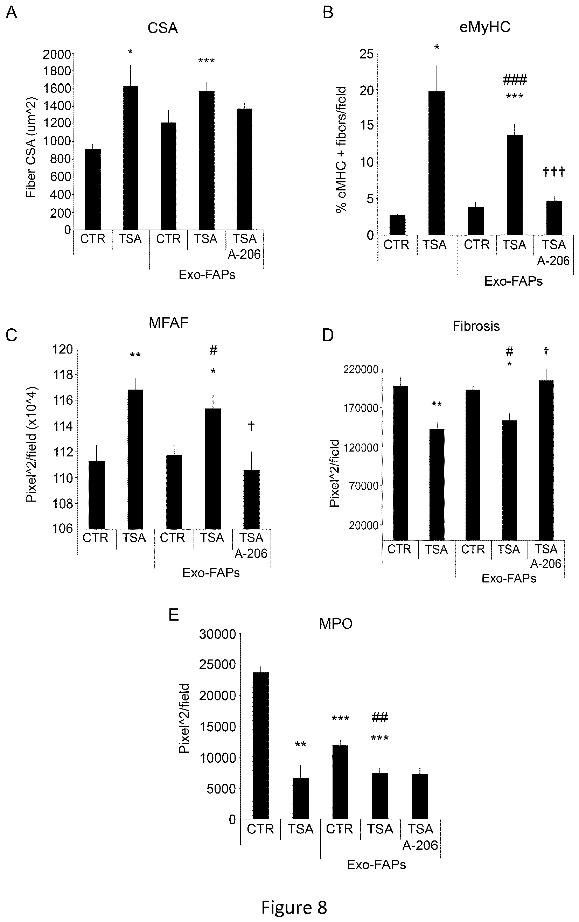

Figure 8: FAPs derived exosomes improve muscle regeneration and miR-206-3p mediates this mechanism. Muscle transversal sections of 1.5 months old mdx mice (n=5 per group) treated daily for 21 days with intra-peritoneal injection of vehicle (CTR) or TSA, or once a week with intramuscular injections (Tibialis Anterior) of exosomes derived from FAPs (Exo-FAPs) exposed or not to TSA *in vivo* (Exo-FAPs CTR, Exo-FAPs TSA) and of Exo-FAPs TSA transfected with antagomiR-206 (TSA A-206). Exosomes were injected every seven days and sacrificed after 21 days of treatment. A) Graph showing the quantifications of cross-sectional area (CSA), measured as µm^2. Statistical significance tested by t-test. Star (*) means significance compared to CTR, *p < 0.01, ***p<0,001 n=5. B) Graph showing the quantifications of muscle regeneration (eMyHC), measured as percentage of eMyHC positive fibers/field. Statistical significance tested by t-test. Star (*) means significance compared to CTR, *p<0,05; ***p < 0.001; hash (#) means significance compared to Exo-FAPs CTR, ###p<0,001; cross (†) indicates significance compared to Exo-FAPs TSA †††p<0,001. n=5 C) Graph showing the quantifications of muscle fiber area fraction (MFAF), measured as pixel^2/field. Statistical significance tested by t-test. Star (*) means significance compared to CTR, *p<0,05; **p < 0.01; hash (#) means significance compared to Exo-FAPs CTR, #p<0,05; cross (†) indicates significance compared to Exo-FAPs TSA †p<0,05 n=5. D) Graph showing the quantifications of fibrosis area, measured as pixel^2/field. Statistical significance tested by t-test. Star (*) means significance compared to CTR, *p<0,05, **p < 0.01; hash (#) means significance compared to Exo-FAPs CTR, #p<0,05; cross (†) indicates significance compared to Exo-FAPs TSA †p<0,05. n=5. E) Graph showing the quantifications of inflammation (MPO), measured as pixel^2/field. Statistical significance tested by t-test. Star (*) means significance compared to CTR, **p < 0.01; ***p<0,001; while hash (#) means significance compared to Exo-FAPs CTR, ##p<0,01. n=5.

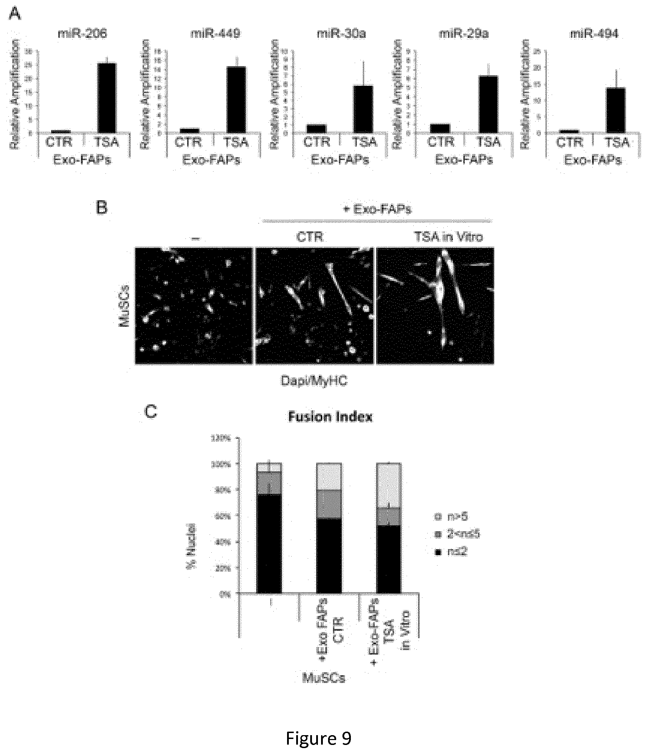

Figure 9: A) Graphs showing the relative expression of microRNAs in exosomes from FAPs *in vitro* treated or not with TSA. B) Representative images of myogenic differentiation of MuSCs assessed by immunostaining for MyHC (white). Nuclei were counterstained with DAPI (grey). MuSCs were cultured alone (-) or with exosomes isolated from FAPs *in vitro* treated or not with TSA. C) Graph showing the fusion index of MuSCs in the condition described in B. n<2 indicates the percentage of nuclei that were MyHC⁻ or MyHC⁺ in mononucleated myotubes. 2 <n< 5 indicates the % of nuclei that were MyHC⁺ inside myotubes containing between 2 and 5 nuclei. n>5 indicates the % of nuclei that were MyHC⁺ inside myotubes containing more than 5 nuclei.

It has been found that FAP-derived exosomes mediate functional interactions with MuSCs, and that exosomal miR content could be pharmacologically modified, in particular by HDACi, for therapeutic purposes.

It has now been found that exosomes derived from FAPs of dystrophic muscles exposed to HDACi promote MuSC activity and compensatory regeneration, while inhibiting fibrosis in mdx mice.

It has been shown that exosomes from FAPs of untreated mdx mice show a reduced ability to promote MuSC activation and differentiation, as compared to exosomes from FAPs of HDACi-treated mdx mice (see for example Figures 3 and 5).

Also, an anti-inflammatory effect has been found for these FAPs-derived exosomes.

FAP-derived exosomes can deliver to MuSCs a cargo of miRs and proteins. The combinatorial effects of the whole exosomal content in miRs and proteins results in the MuSC activation and regeneration of new fibers.

Among these miRNAs, miR-206 is particularly relevant for its effects on MuSCs *ex vivo* and on regeneration of dystrophic muscles *in vivo.*

Indeed, exosomes purified from FAPs transfected with antagomiR-206 showed a reduced ability to promote MuSC-mediated formation of multinucleated myotubes, as compared to exosomes purified from FAPs isolated from vehicle (TSA), as well as to FAPs from untreated (CTR) mdx mice (see for example Figure 5A-C). Thus, miR-206 is an important component of the FAP-derived exosomes that support MuSC differentiation ability *ex vivo.* In particular, our study reveals that FAPs from dystrophic muscles turn into an additional cellular source of miR-206 once exposed to HDACi.

The present invention also provides the following advantages:
- a more selective and targeted activity with respect to systemic administration of HDAC inhibitors; this avoids or lessens the probability of collateral effects, toxicity and off-targets activities which are typical of HDAC inhibitors;
- avoidance of pharmacological interactions with other drugs used in the treatment of muscular dystrophies, such as for example steroids which may interact with HDAC inhibitors;
- broad dosage spectrum since dose-limiting toxicities, as observed with small molecule drugs such as HDAC inhibitors, are less likely to occur;
- more localized activity thanks to local and targeted administration of the exosomes, which allows to restrict and focus the treatment to specific muscles. This is particularly advantageous in cases wherein the subject has only few muscles still intact.

The vesicle of the invention can therefore be advantageously used in the treatment of a subject affected by muscular dystrophy.

### Exosomes

Secreted by a wide range of cell types, exosomes are lipid bilayer vesicles that are enriched in a variety of biological factors, including cytokines, growth factors, transcription factors, lipids, and coding and non-coding nucleic acids.

Exosomes are found in blood, urine, amniotic fluid, interstitial and extracellular spaces. These exocytosed vesicles of endosomal origin can range in size between 30-200 nm, including sizes of 40-100 nm, and possess a cup-shaped morphology, as revealed by electron microscopy. Their initial formation begins with inward budding of the cell membrane to form endosomes, which is followed by invagination of the limiting membrane of late endosomes to form multivesicular bodies (MVB). Fusion of the MVB with the plasma membrane results in the release of the internal vesicles to the extracellular space, through the formation of vesicles thereafter known as exosomes.

The "cargo" contents of exosomes reflect their parental cellular origin, as containing distinct subsets of biological factors in connection with their parent cellular origin, including the cell regulatory state of the parental cells when formed. The rich biological milieu of different proteins, including cytokines and growth factors, lipids, coding and noncoding RNA molecules, within exosomes are all necessarily derived from their parental cells. In addition to containing a rich array of cytosolic derivatives, exosomes further express the extracellular domain of membrane -bound receptors at the surface of the membrane.

The described encapsulation and formation processes necessarily create heterogeneity in exosome compositions based on parental cellular origin and regulatory state at time of formation. Nevertheless, generic budding formation and release mechanisms establish a common set of features as a consequence of their origin, such as endosome-associated proteins (e.g., Rab GTPase, SNAREs, Annexins, and flotillin), proteins that are known to cluster into microdomains at the plasma membrane or at endosomes (four transmembrane domain tetraspanins, e.g., CD63, CD81, CD82, CD53, and CD37), lipid raft associated proteins (e.g., glycosylphosphatidylinositol- anchored proteins and flotillin), cholesterol, sphingomyelin, and hexosylceramides, as examples. In addition to these core components reflecting their vesicle origin, a critical property of exosomes is a demonstrated capability to contain both mRNA and microRNA associated with signaling processes, with both cargo mRNA being capable to translation in recipient cells, or microRNA functionally degrading target mRNA in recipient cells. Other noncoding RNAs, capable for influencing gene expression, may also be present in exosomes. While the processes governing the selective incorporation of mRNA or microRNA populations into exosomes is not entirely understood, it is clear that RNA molecules are selectively, not randomly incorporated into exosomes, as revealed by studies reporting enrichment of exosome cargo RNAs when compared to the RNA profiles of the originating cells. Given the growing understanding of how such RNA molecules play a role in disease pathogenesis and regenerative processes, the presence of RNA molecules in exosomes and apparent potency in affecting target recipient cells suggests critical features that can be deployed in therapeutic approaches.

Importantly, the natural bilayer membrane encapsulation of exosomes provides a protected and controlled internal microenvironment that allows cargo contents to persist or migrate in the bloodstream or within tissues without degradation. Their release into the extracellular environment allows for interaction with recipient cells via adhesion to the cell surface mediated by lipid-ligand receptor interactions, internalization via endocytic uptake, or by direct fusion of the vesicles and cell membrane. These processes lead to the release of exosome cargo content into the target cell.

The net result of exosome-cell interactions is modulation of genetic pathways in the target recipient cell, as induced through any of several different mechanisms including antigen presentation, the transfer of transcription factors, cytokines, growth factors, nucleic acid such as mRNA and microRNAs. In the stem cell context, embryonic stem cell (ESC)- derived exosomes have been demonstrated to shuttle/transfer mRNA and proteins to hematopoietic progenitors. Other studies have shown that adult stem cell-derived exosomes also shuttle selected patterns of mRNA, microRNA and pre-microRNA associated with several cellular functions involved in the control of transcription, proliferation and cell immune regulation.

### Isolation and Preparation of Exosomes

Exosome isolation relies on exploiting their generic biochemical and biophysical features for separation and analysis.

For example, differential ultracentrifugation has become a leading technique wherein secreted exosomes are isolated from the supernatants of cultured cells. This approach allows for separation of exosomes from non-membranous particles, by exploiting their relatively low buoyant density. Size exclusion allows for their separation from biochemically similar, but biophysically different microvesicles, which possess larger diameters of up to 1,000 nm. Differences in flotation velocity further allows for separation of differentially sized exosomes. In general, exosome sizes will possess a diameter ranging from 30-200 nm, including sizes of 40-100 nm. Further purification may rely on specific properties of the particular exosomes of interest. This includes, for example, use of immunoadsorption with a protein of interest to select specific vesicles with exoplasmic or outward orientations.

Among current methods (differential centrifugation, discontinuous density gradients, immunoaffmity, ultrafiltration and high performance liquid chromatography (HPLC)), differential ultracentrifugation is the most commonly used for exosome isolation. This technique utilizes increasing centrifugal force from 2000xg to 10,000xg to separate the medium- and larger-sized particles and cell debris from the exosome pellet at 100,000xg. Centrifugation alone allows for significant separation/collection of exosomes from a conditioned medium, although it is insufficient to remove various protein aggregates, genetic materials, particulates from media and cell debris that are common contaminants. Enhanced specificity of exosome purification may deploy sequential centrifugation in combination with ultrafiltration, or equilibrium density gradient centrifugation in a sucrose density gradient, to provide for the greater purity of the exosome preparation (flotation density I .I-1.2g/ml) or application of a discrete sugar cushion in preparation.

Importantly, ultrafiltration can be used to purify exosomes without compromising their biological activity. Membranes with different pore sizes - such as 100 kDa molecular weight cut-off (MWCO) and gel filtration to eliminate smaller particles - have been used to avoid the use of a nonneutral pH or non-physiological salt concentration. Currently available tangential flow filtration (TFF) systems are scalable (to >10,000L), allowing one to not only purify, but concentrate the exosome fractions, and such approaches are less time consuming than differential centrifugation. HPLC can also be used to purify exosomes to homogeneouslysized particles and preserve their biological activity as the preparation is maintained at a physiological pH and salt concentration. Other chemical methods exploit differential solubility of exosomes for precipitation techniques, addition to volume-excluding polymers (e.g., polyethylene glycols (PEGs)), possibly combined additional rounds of centrifugation or filtration. For example, precipitation reagents, ExoQuick^{®} or Total Exosome Isolation Reagents-TEIR^{®}, can be added to conditioned cell media to quickly and rapidly precipitate a population of exosomes. Flow field-flow fractionation (F1FFF) is an elution-based technique that is used to separate and characterize macromolecules (e.g., proteins) and nano- to micro-sized particles (e.g., organelles and cells) and which has been successfully applied to fractionate exosomes from culture media.

In a particular embodiment of the present invention, the exosomes are isolated using the technique disclosed in Tucciarone et al. 2018.

The exosomes are obtained by a method including a) providing a population of fibro-adipogenic progenitors (FAP) cells which have been exposed to HDAC inhibitors wherein said histone deacetylase inhibitor is selected from the group consisting of Trichostatin A,MS-275, suberanilohydroxamic acid (SAHA) and Givinostat and b) isolating a plurality of exosomes from said cells.

Said fibro-adipogenic progenitors (FAP) cells preferably originate from dystrophic muscles. This means that, in an embodiment, such cells have been derived from the dystrophic muscle of a subject. For example, they can be isolated from the muscle of a dystrophic subject. After their sampling from the dystrophic muscle, they can be expanded *in vitro* for limiting passages and/or stored, for example in liquid nitrogen, for future usage, upon defrosting and culture. Such techniques are all within the common knowledge of the skilled person.

Said cells are isolated by the common technique of fluorescence activated cell sorting (FACS) from a dystrophic animal, in particular mice.

Said cells are isolated from the biopsy of human subjects affected by a dystrophic disease, in particular boys. Preferably, said subject is at early stages of the disease, in particular in a stage in which the regenerative potential is not exhausted and the muscle is able to respond to HDACi. The person skilled in the art is able to identify the human or animal subject suitable for isolation of the FAP cells based on the common general knowledge in this clinical field.

The exosomes of the invention are obtained by a method including a) providing a population of fibro-adipogenic progenitor (FAP) cells isolated from a dystrophic muscle of a subject, said subject being previously treated with HDAC inhibitors, wherein said histone deacetylase inhibitor is selected from the group consisting of Trichostatin A,MS-275, suberanilohydroxamic acid (SAHA) and Givinostat and b) isolating a plurality of exosomes from said cells.

In a first step, therefore, said FAPs cells are isolated from a dystrophic subject to which HDAC inhibitors wherein said histone deacetylase inhibitor is selected from the group consisting of Trichostatin A,MS-275, suberanilohydroxamic acid (SAHA) and Givinostat were previously administered in an amount currently used in therapy.

HDAC inhibitors are well known compounds that inhibit histone deacetylases. The latter are a class of enzymes that remove acetyl residues from post-translationally acetylated lysine residues, found in histones and non-histone proteins. 11 different, Zn-dependent and 7 non-Zn dependent HDACs are known. HDAC inhibitors are selected from the group consisting of Trichostatin A, MS-275, suberanilohydroxamic acid (SAHA) and Givinostat Trichostatin A is an inhibitor of the Zn dependent HDACs, . MS-275, SAHA or Givinostat (Minetti et al., 2006, Colussi et al. 2010, Consalvi et al. 2013) were described to be active in the treatment of animals affected by muscular dystrophy. In a phase I/II clinical trial, Givinostat was shown to positively affect the histology of the muscles of DMD patients after one year of treatment and the compound is presently undergoing phase III clinical trials (Bettica et al 2016). The subject to be treated can be a human or an animal dystrophic subject, for example a mouse.

For example, FAP cells can be isolated as TER119^{neg} /CD45^{neg} /CD31^{neg} /α7INTEGRIN^{neg} /SCA-1^{pos} cells, using a procedure described in Mozzetta et al. (2013).

Then, exosomes are isolated from said cells.

The exosomes of the invention are obtained by a method including a) providing a population of fibro-adipogenic progenitor (FAP) cells originating, for example isolated, from a dystrophic muscle, b) exposing said cells *in vitro* to HDAC inhibitors wherein said histone deacetylase inhibitor is selected from the group consisting of Trichostatin A,MS-275, suberanilohydroxamic acid (SAHA) and Givinostat and c) isolating a plurality of exosomes from said cells.

The cells obtained after step a) or b) are expanded and stored *in vitro* until the next step is performed.

FAPs can be isolated from a dystrophic subject, for example an animal dystrophic subject, for example a mouse. FAP cells can be isolated as TER119^{neg} /CD45^{neg} /CD31^{neg} /α7INTEGRIN^{neg} /SCA-1^{pos} cells, using a procedure described in Mozzetta et al. (2013). They are cultured in appropriate culture media according to the common knowledge in the field. Preferably, they are cultured in serum free cell culture medium 24 hours before exosome isolation.

In a second step, said cells are exposed *in vitro* to HDAC inhibitors.

For example, cells can be exposed to HDAC inhibitors for 15 hours before changing the medium with exosome free medium. An incubation of 24 hours may follow. The above mentioned HDAC inhibitors can be used.

Finally, exosomes are isolated from the FAP cells.

Typically, the exosomes are isolated from the supernatants of the population of cells using suitable exosome isolation reagents, such as Total Exosome Isolation^{™} precipitation reagent (TEIR), or by differential ultracentrifugations (UC) (Thery et al., 2006; Van Deun et al., 2014).

For an exemplary exosome isolation method reference can be made to Tucciarone et al., 2018.

Isolating exosomes from the population of cells includes centrifugation of the cells and/or media conditioned by the cells. In several embodiments, ultracentrifugation is used. In several embodiments, isolating a plurality of exosomes from the population of cells is via size-exclusion filtration. In other embodiments, isolating a plurality of exosomes from the population of cells includes use of discontinuous density gradients, immunoaffinity, ultrafiltration and/or high performance liquid chromatography (HPLC).

Isolating a plurality of exosomes from the population of cells includes use of one or more capture agents to isolate one or more exosomes possessing specific biomarkers or containing particular biological molecules. In one embodiment, one or more capture agents include at least one antibody. For example, antibody immunoaffmity recognizing exosome-associated antigens is used to capture specific exosomes. In other embodiments, the at least one antibody are conjugated to a fixed surface, such as magnetic beads, chromatography matrices, plates or microfluidic devices, thereby allowing isolation of the specific exosome populations of interest. In other embodiments, isolating a plurality of exosomes from the population of cells includes use of one or more capture agents that is not an antibody. This includes, for example, use of a "bait" molecule presenting an antigenic feature complementary to a corresponding molecule of interest on the exosome surface, such as a receptor or other coupling molecule. In one embodiment, the non-antibody capture agent is a lectin capable of binding to polysaccharide residues on the exosome surface.

All these techniques are well known in the field and can be applied by the skilled person according to his general knowledge.

For example, EVs can be isolated and quantified according to the previously published method by Thery et al. (2006). Also, for example, they can be isolated by the method disclosed in Tucciarone et al. 2018.

Exosomes are typically about 30 nm to about 200 nm in diameter, preferably from 100 to 150 nm.

Exosomes typically express a biomarker, such as tetraspanins (CD9, CD63, CD81) Alix, flotillin-1 and Tsg101 (Théry et al., 2002).

The exosome contains microRNAs. In various embodiments, said microRNA can be one or more microRNAs selected from the group consisting of: miR-206-3p, miR-542-5p, miR-449a-5p, miR-342-3p, miR-320-3p, miR-192-5p, miR-423-5p, miR-376a-3p, miR-145a-5p, miR-224-5p, miR-30a-5p, miR-494-3p, miR-29a-3p, miR-7b-5p.

In several embodiments, the exosome is enriched in at least one of said miRNAs. In a preferred embodiment, said miRNA is miR-206.

Enrichment can be measured in absolute or relative quantities, such as when compared to a standardized dilution series.

The exosomes of the invention are able to promote MUSCs activity and regeneration and to inhibit fibrosis in the dystrophic muscle.

It is also an object of the invention a composition comprising a plurality of said exosomes.

A chief goal of developing exosome-based therapy is the creation of "cell-free" therapies, wherein the benefits of cellular therapeutics can be provided with reduced risks or in scenarios in which cell therapy would be unavailable.

Cellular exosomes produced by FAPs exposed to HDAC inhibitors may allow for production and delivery of growth factors, transcription factors, cytokines and nucleic acids in a manner that not only ameliorates progression of the disease, but repairs and regenerates disease and/or dysfunctional tissue.

In particular, stem cell-derived exosomes are likely to be less immunogenic than parental cells. The possibility of replacing the administration of live cells with secreted exosomes, mitigates many of the safety concerns and limitations associated with the transplantation of viable cells. In addition, exosome encapsulation of bioactive components in lipid vesicles allows protection of contents from degradation in vivo, thereby potentially negating obstacles often associated with delivery of soluble molecules such as cytokines, growth factors, transcription factors and RNAs, while potentially allowing for increased concentrations to be provided. Particularly for chronic conditions, such as DMD, repeated and sustained delivery to patients may maximize the potential for regeneration and repair of diseased and/or dysfunctional tissue, in a manner that would be difficult or unsafe with a cell- based therapy.

### Medical uses

Object of the invention is said extracellular vesicle or exosome for use in the treatment of a muscular dystrophy.

Different categories and types of muscular dystrophies are known. They differ mainly in which muscles are primarily affected, the degree of weakness, how fast they worsen, and when symptoms begin.

For example, said muscular dystrophy can be selected from the group consisting of: Duchenne Muscular Dystrophy, Becker Muscular Dystrophy, Limb-Girdle Muscular Dystrophy and other forms of muscular dystrophies and/or muscle disorders sharing common pathogenesis with the forms indicated above.

Said dystrophy is preferably Duchenne Muscular Dystrophy (DMD).

The affected subject can be in any clinical phase of the disease, e.g. an early or tardive phase. Preferably, the subject to be treated is a young subject.

In an embodiment, said subject is in an advanced phase of the disease. This subject typically has only few muscles still partially intact, such as the muscles not yet damaged by the disease, typically forearm and hand muscles, or the muscles necessary to keep the subject alive, such as diaphragm. The exosomes of the invention can be advantageously used in these subjects, in particular because they can be administered in a selective manner, for example by local injection, to the muscles which still retain some activity thus allowing to treat subjects which can not generally be treated with known therapies due to the severity of the disease.

The skilled person in the field knows how to determine the suitable dose in view of the desired application and his general knowledge in the medical and pharmaceutical field and by carrying out routine experimentation.

Another object of the present invention is a pharmaceutical composition containing at least one of said extracellular vesicle or exosome, typically in an amount such as to produce a significant therapeutic effect, together with pharmaceutically acceptable vehicles and/or excipients.

Such pharmaceutical compositions can be prepared by conventional methods and techniques which are common practice in the pharmaceutical industry, such as, for example, those illustrated in Remington's Pharmaceutical Science Handbook, Mack Pub. N.Y. - last edition.

The composition according to the present invention contains, along with the exosome as active ingredient, at least one pharmaceutically acceptable vehicle or excipient. These may be particularly useful formulation coadjuvants, e.g. solubilising agents, dispersing agents, suspension agents, and emulsifying agents.

The pharmaceutical composition according to the invention comprising said exosomes can also contain one or more further active ingredients, for example active ingredients of common use in the treatment of muscular dystrophies.

According to the administration route chosen, the compositions will be in solid or liquid form, suitable for oral, parenteral or topical administration.

Average quantities of the active agent may vary and in particular should be based upon the recommendations and prescription of a qualified physician.

Generally, the compounds of this invention are administered in a "therapeutically effective amount". The amount of the compound actually administered will typically be determined by a physician, in the light of the relevant circumstances, including the condition to be treated, the chosen route of administration, the actual compound administered, drug combination, the age, body weight, and response of the individual patient, the severity of the patient's symptoms, and the like. For any compound, the therapeutically effective dose can be estimated initially either in cell culture assays or in animal models, usually mice, rats, guinea pigs, rabbits, dogs, or pigs. The animal model may also be used to determine the appropriate concentration range and route of administration. Such information can then be used to determine useful doses and routes for administration in humans. In calculating the Human Equivalent Dose (HED) it is recommended to use the conversion table provided in Guidance for Industry and Reviewers document (2002, U.S. Food and Drug Administration, Rockville, Maryland, USA).

Generally, an effective dose will be from 0.01 mg/kg to 100 mg/kg, preferably 0.05 mg/kg to 50 mg/kg. For any compound, the therapeutically effective dose can be estimated initially either in cell culture assays or in animal models, usually mice, rats, guinea pigs, rabbits, dogs, or pigs. The precise effective dose for a human subject will depend upon the severity of the disease state, general health of the subject, age, weight, and gender of the subject, diet, time and frequency of administration, drug combination(s), reaction sensitivities, and tolerance/response to therapy. This amount can be determined by routine experimentation and is within the judgement of the clinician.

Compositions may be administered individually to a patient or may be administered in combination with other agents, drugs or hormones. For example they can be used in combination with further active ingredients.

The medicament may also contain a pharmaceutically acceptable carrier, for administration of a therapeutic agent. Such carriers include antibodies and other polypeptides, genes and other therapeutic agents such as liposomes, provided that the carrier does not induce the production of antibodies harmful to the individual receiving the composition, and which may be administered without undue toxicity.

Suitable carriers may be large, slowly metabolised macromolecules such as proteins, polysaccharides, polylactic acids, polyglycolic acids, polymeric amino acids, amino acid copolymers and inactive virus particles.

A thorough discussion of pharmaceutically acceptable carriers is available in Remington's Pharmaceutical Sciences (Mack Pub. Co., N.J. 1991).

Pharmaceutically acceptable carriers in therapeutic compositions may additionally contain liquids such as water, saline, glycerol and ethanol.

Additionally, auxiliary substances, such as wetting or emulsifying agents, pH buffering substances, and the like, may be present in such compositions. Such carriers enable the pharmaceutical compositions to be formulated as tablets, pills, dragees, capsules, liquids, gels, syrups, slurries, suspensions, and the like, for ingestion by the patient.

Once formulated, the compositions of the invention can be administered directly to the subject. The subjects to be treated can be animals; in particular, human subjects can be treated.

The medicament of this invention may be administered by any number of routes including, but not limited to, oral, intravenous, intramuscular, intraarterial, intramedullary, intrathecal, intraventricular, transdermal or transcutaneous applications, subcutaneous, intraperitoneal, intranasal, enteral, topical, sublingual, intravaginal or rectal means. Intramuscular injection is a preferred administration route.

The administration regime, dosage and posology will be determined by the physician according to his experience, the disease to be treated and the patient's conditions.

The compositions for oral administration may take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include refilled, pre-measured ampoules or syringes of the liquid compositions or pills, tablets, capsules or the like in the case of solid compositions. In such compositions, the compound of the invention is usually a minor component (for example from about 0.1 to about 50% by weight or preferably from about 1 to about 40% by weight) with the remainder being various vehicles or carriers and processing aids helpful for forming the desired dosing form. Dosage treatment may be a single dose schedule or a multiple dose schedule.

A process for the preparation of said pharmaceutical compositions characterized by mixing one or more exosomes with suitable excipients, stabilizers and/or pharmaceutically acceptable diluents.

It is also an object of the invention such pharmaceutical composition for use as a medicament, in particular in the treatment of muscular dystrophies. More in particular, DMD can be treated by such pharmaceutical compositions.

It is known in the art, that HDAC inhibitors such as Vorinostat have a bell-shaped dose response curve in a preclinical model of DMD (Colussi C et al., Proteomics Clin. Appl. 2010, 4, 71-83). This renders the identification of the optimal dose that can be used to treat a patient affected by muscular dystrophy particularly challenging. Colussi et al proposed to use serum biomarkers. The biomarkers that they have identified, however, are modulated to a very minor extent and their usefullness in the clinic to guide dose selection is therefore questionable. In addition the mechanistic link between the serum biomarkers identified by Colussi et al with the disease is not established.

In contrast, the extracellular vesicles of the present invention are clearly linked to the mechanism by which HDAC inhibitors affect disease progression of DMD in the preclinical model and are robustly upregulated in muscle tissue upon pharmacological treatment.

In particular, as already mentioned, the treatment with HDAC inhibitors changes the content of exosomes released from FAP cells. Therefore, the analysis of the content of said exosomes allows to understand if the treatment has been successful, in particular if the inhibitor was administered at the correct dosage.

Exosomes released from FAPs are firstly obtained from the muscle or a biological fluid of a subject, said subject having undergone treatment with an HDAC inhibitor. Subsequently, such exosomes are analyzed in their content to assess if the treatment was successful. For instance, in situ staining of exosomal miRs that are upregulated by HDACi can be a suitable tool to measure markers of efficacy of HDACi treatment from histological analysis of muscle sections (Giordani et al. 2014) or patient muscle biopsies (Bettica et al. 2016).

Likewise, the same procedure can be used to assess the status of muscles in DMD patients to estimate the stage of disease progression and predict response to pharmacological interventions.

Therefore, the extracellular vesicle of the invention can also be used *ex vivo* for assessing the status of muscles in DMD patients, for example to estimate the stage of disease progression, and to predict response to pharmacological interventions.

The following examples further illustrate the invention.

### EXAMPLES

### Materials and methods

### METHOD DETAILS

### Mice and animal procedures

Animals were used at the specified age and treated for the indicated periods with daily intra peritoneal injections of Trichostatin A, TSA (0.6 mg/kg/day; #T8552, Sigma), dissolved in in phospate-saline solution or in phospate-saline alone as vehicle control (CTR). Muscle injury was performed by intramuscular injection in Tibliais Anterior (TA) of Cardiotoxin -CTX- (10 µM, 10 mg/mL) (#L8102, Lotaxan Valence, France, http://www.latoxan.com), 3 days before mice sacrifice.

FAPs-derived exosomes at a final concentration of 10 µg in 20 µL of PBS1x (0.5ug/µl) (#14190-1444, Gibco by life technologies) and its vehicle of control (20 µl PBS1x) were injected 3 times in left TA of young C57BL6/mdx mice: every 7 days for 21 days.

### Histology and in situ hybridization

The Tibialis anterior muscles were snap frozen in liquid nitrogen-cooled isopentane and then cut transversally with a thickness of 7 µm.

For Masson Trichrome staining to analyze fibrotic tissue, muscle cryo-sections were fixed for 20' at 56°C in Bouin's Solution (#HT10132, Sigma) and then stained in Working Weigert's Iron Hematoxilin solution for 5 min (#HT1079, Sigma), washed in running tap water for 5 min and stained in Biebrich Scarlet-Acid Fucsin for 5 min (#HT151, Sigma). Sections were rinsed in de-ionized water and re-fixed in freshly made Phosphomolybdic/Phosphotungstic/dH₂O (1:1:2) acid solution for 8 min (#HT153, #HT152, Sigma), and then they are stained in Aniline Blue solution for 5 min (#HT154, Sigma) and in acid acetic 1% for 2 min (#27221, Sigma). The slides were dehydratated in ethanol (#02860, Sigma) and xilene (#X1040, Sigma) and mounted with EUKITT (#03989, Sigma), then visualized using a Nikon Eclipse 90i: collagen fibers are stained in blue, the nuclei stained in black and the muscle tissue is stained in red. miRNA *in situ* hybridization was performed as described by Pena (2009) in formaldehyde and carbodiimide (EDC)-fixed TA cryo-sections (0,16M 90 min at RT,_#25952-53-8, Merck KGaA). After washes with 0,2% glycine (#G8898, Sigma) and TBS, cryo-sections were acetylated using 0,1 M triethanolamine and 0,25% acetic anhydride for 25 min at RT (respectively #90275, #A6404, Sigma). This steps are followed by a pre-hybridization using 2X of SSC, 25% formamide (#F9037, Sigma); 0,2% Triton (#X100, Sigma) 30 min at RT and by the over night hybridization at 4°C with the has-miR206 probe (10 pmol, #18100-01, exiqon) dissolved in a solution of 50% formamide, 250µg/ml tRNA (#R1753, Sigma), 200µg/ml SSDNA (#D7656, Sigma), 10% dextran sulphate (#D8906, Sigma) and 2X SSC. The hybridization was followed by specific washes with SSC to eliminate non specific binding of probe (5X SSC 5min at RT, 1X SSC 15 min at 45°C, 2%BSA in 0,2X SSC 15min at 4°C, 2X SSC 5min at RT, TNbuffer 10 min at RT and TNT buffer 15' at RT) and by the incubation of cryo-section using anti-digoxigenin-ap fab fragments (1/100, #11093274910, Roche) dissolved in TNbuffer for 2 hours at RT. To reveal the miRNA probe specific binding cryo-sections, covered from light, were incubating over night at 4°C with 0,375 mg/ml of NBT and 0,188 mg/ml BCIP dissolved in a solution of TMNbuffer (respectively #11383213001 and #11383221001, Roche).

TNbuffer is composed of 0,1 M Tris-HCL (#T1503, Sigma) and 0,15 M Nacl (#S3014, Sigma) at pH 7,5; TNTbuffer is TNbuffer with 0,1% Tween (#P1379, Sigma) while TMNbuffer is composed of 0,1 M Tris-HCL, 0,005M MgCl₂ (#M8366, Sigma), 0,5 M NaCl and 2mM of Levamisole (#L9756, Sigma).

### Isolation of FAPs and Satellite cells (MuSCs).

FAP cells were isolated as TER119^{neg}/CD45^{ne3}/CD31^{neg}/· 7INTEGRIN^{neg}/SCA-1^{pos} cells, MuSCs were isolated as TER119^{neg}/CD45^{neg}/CD31^{neg}/· 7INTEGRIN^{pos}/SCA-1^{neg} cells using a procedure described in Mozzetta et al. (2013).

Briefly, hind limb muscles for each mouse were minced and put into a 15 mL tube containing 4 mL of HBSS (#24020-091, GIBCO) BSA (0.2%, #A7030, Sigma) and
10 Units/ml Penicillin and 10 µg/ml streptomycin (P/S), 2 mg/ml Collagenase A (#10103586001, Roche), 2.4U/ml Dispase II (#04942078001, Roche), DNaseI 10 mg/ml (#11284932001, Roche) at 37°C under gentle agitation for 1hr and 30 min.

The supernatants were filtered through a 100· m, 70· m and 40· m cell strainers (#08-771-19, #08-771-2, #08-771-1, BD Falcon). Cells were spun for 15 min at 300 g at 4°C, the pellets were re-suspended in 0.5 mL of HBSS 1× containing DNase I and incubated with antibodies on ice for 30 min. The following antibodies were used: CD45-eFluor 450 (1/50, #48-0451-82, Leukocyte Common Antigen, Ly-5, eBiosciences), CD31- eFluor 450 (1/50, PECAM-1, #48-0311-82, eBioscience), TER-119- eFluor 450 (1/50, clone TER-119, #48-5921-82, eBiosciences), Sca1-FITC (1/50, Ly-6A/E FITC, clone D7, #11-5981-82, eBioscience), Itga7-649 (1/500, AbLab #67-0010-01). HBSS was added and cells were spun for 5 min at 300 g at 4°C to stop the reaction. The cells were re-suspended in HBSS containing 1% DNaseI and were isolated based on size, granulosity and fluorophores levels using a FACS MoFlo HS Cell Sorter Dako Cytomation (BD) and analyzed using FlowJo.

### Cell culture

MuSCs and FAPs were cultured after sorting directly in culture media: for MuSCs: 20% FBS (#16000044, GIBCO), 10% HS (#26050-070, GIBCO), 1% Penicillin-Streptomycin (#15140, GIBCO), 1% Chicken Embryo Extract (CEE, #CE-650-F, Seralab) in DMEM + Pyruvate (#41966, GIBCO) and for FAPs: BIOAMF-2 (Biological Industries). MuSCs were plated at low density on regular cell culture dishes coated with Gelatin 0.1% (#07903, Stemcell).

FAP cells were cultured in Bioamf-2 at high density (200.000 cells in 10 cm-well plate) for exosome isolation, at low density: 40000 or 10000 cells respectively in 6 or 24 well dishes for co-culture experiments.

To isolate exosomes, FAP cells reached 80-90% of confluence then, the medium was replaced with DMEM (+ pyruvate + 4.5 g/l glucose + glutamate) serum free for 24 hr.

### Transwell co-culture experiments

MuSCs and FAP cells were co-cultured by using inserts with 1.0 µm porous membrane to avoid direct contact between populations.

Cell culture inserts with 1.0-· m pore (#353102, #353104, Falcon) and 6 or 24 -well culture plates (BD Bioscience) were used for transwell co-culture. Freshly sorted MuSCs were plated in the bottom of the plate, while FAP cells were plated on the upper insert.

### Single fibers isolation

Single fibers were isolated using Pasut (2013) and Moyle's protocol (2014) from tibialis anterior, extensor digitorum longus, gastrocnemius and soleus muscles of C57BL6J-WT mice and cultured in proliferating medium (GM1: DMEM + pyruvate +4.5 g/l glucose + glutamate, 10% horse serum (HS), 0.5% Chicken Embryo Extract) for 24 hours, then exposed for the next 24 hrs to GM1 conditioned media derived from culture of FAPs (MEDIA-FAPs) isolated from young mdx mouse treated for 15 days with vehicle (CTR) or mdx mice treated with TSA (TSA) or media isolated from FAPs CTR and TSA pre-treated with the GW4869 to inhibit exosome biogenesis. In a parallel, independent experiment, single fibers were exposed to exosomes (EXO-FAPs) purified from MEDIA-FAPs and added to GM1 for 24 hours.

### Cell treatments

The transfection of FAPs with pCT-CD63-GFP plasmid (#CYTO120-PA-1, System Biosciences) and with a GFP-control vector (mock) in cell culture inserts was accomplished using Lipofectamine 2000 (#12566014, Thermofisher Scientific) 6 hours before MuSCs co-colture. Transwell co-cultures were maintained in GM2 medium for 24hrs and then harvested for GFP-analyses.

To decrease FAP-exosomes release, GW4869 (10 uM, #D1692, Sigma) was added to FAPs culture 30' before the co-culture setting with MuSCs. To wash away any residual trace of GW4869, FAPs media was refreshed right before co-culture.

For AntagomiR FAPs isolated from mdx mice TSA treated, were transfected with AntagomiR-206 (mmu-miR-206-3p miRCURY LNA^{™} microRNA inhibitor, Exiqon) and mock (miRCURY LNA^{™} microRNA inhibitor control #199006, Exiqon) using Dharmafect3.0 (#T2003-03, Thermo Fisher Scientific) according to manufacturer instructions; after 6 hours the medium was replaced with DMEM serum free for 24 hours before exosome isolation and purification.

For Drosha SiRNA treatments, *in vivo* TSA treated FAPs on transwell were transfected with commercially available Drosha siRNA using Dharmafect3.0 (Dharmacon) according to manufacturer instructions; after 6 hours FAPs were put in co-culture with MuSCs.

To stain FAPs derived exosomes, FAP cells were incubated with the lipidic dye PKH-67 (#P7333, Sigma) according to manufacturer instructions prior to transwell co-culture with MuSCs.

In ex-vivo experiments FAP-derived Exosomes (10· g) isolated by TEIR were put in culture with MuSCs or with myofibers in the cell culture media.

### Immunofluorescence

For immunofluorescence analysis, cryo-sections and cells were fixed in 4% PFA for 10 min and permeabilized with 100% cold acetone (#32201, Sigma) for 6 min at -20°C or 100% cold Methanol (#32213, Sigma) for 6 min at -20° or with 0,25% Triton for 15 min at RT. Muscle sections were blocked for 1h with a solution containing 4% BSA (#A7030, Sigma) in PBS. The primary antibodies incubation were performed O.N. at 4°C and then the antibody binding specificity was revealed using secondary antibodies coupled to Alexa Fluor 488, 594, or 647 (Invitrogen). Sections were incubated with DAPI in PBS for 5 minutes for nuclear staining, washed in PBS, and mounted with glycerol 3:1 in PBS. The primary antibodies used for immunofluorescences are: rabbit anti-Laminin (1/400, #L9393, Sigma); rat anti-SCA1 (1/100, #11-5981-82 Ly-6A/E FITC, eBioscience,); mouse anti-eMyHC (1/20, #F1.652, Developmental Studies Hybridoma Bank, DSHB, http://dshb.biology.uiowa.edu/F1-652); mouse anti-MF20 (1:20, Developmental Studies Hybridoma Bank, DSHB, http://dshb.biology.uiowa.edu/MF-20), mouse anti-PAX7 (1/10, Developmental Studies Hybridoma Bank, DSHB, http://dshb.biology.uiowa.edu/PAX7), rabbit anti-MyoD-318 (#SC760, Santa Cruz Biotechnology), EDU (#C10350, Invitrogen), rat anti-CD63 PE (#143904, clone NUG-2, Biolegend) and Myeloperoxidase/MPO (1/100 #MAB3174, R&D).

### Exosomes

### -Exosome Isolation

Exosomes were isolated from FAPs serum free cell culture medium in parallel with Total exosomes isolation reagent -TEIR- (#4478359, Invitrogen by Thermo Fisher Scientific) according to manufacturer instructions. EVs were isolated and quantified according to a previously published method (Thery et al., 2006). This isolation method included a penultimate centrifugation step in Eppendorf polypropylene conical tubes (10,000 × g for 30 min at 4°C, in Eppendorf rotor F-34-6-38) that allowed the removal/isolation of larger microvesicles. Subsequently, nano-sized EVs, comprised mainly of exosomes, were pelleted in Beckman Coulter polypropylene open top tubes (118,000 × g for 70 min at 4°C, in Beckman rotor SW28). After washing, the pellet was resuspended either in RIPA buffer or in PBS, for further immunoblotting or biophysics and molecular analyses, respectively. To estimate the amount of secreted vesicles, we quantified and compared the total protein content of the vesicle lysates using the BCA assay.

Exosomes were purified from tibialis anterior gently dissociated in serum-free DMEM (24 h). Cell debris and organelles were eliminated by centrifugation at 2,000g for 20 min and exosomes were isolated using TEIR.

### -Exosome characterization

Exosome size distribution was determined by Dynamic Light Scattering (DLS) measurements. Collected exosome samples were diluted to a final concentration of 15µg/ml total protein content in order to avoid multiple scattering artifacts. Static and dynamic light scattering measurements were performed at 20°C by using a Brookhaven Instruments BI-9000 correlator and a solid-state laser tuned at *λ₀* = 532 nm. Scattered intensity autocorrelation functions *g2(t)* have been analyzed by using multiple gamma functions for the diffusion coefficient and therefore by using the classic Stokes-Einstein relation to determine the size distribution P(D) of vesicles, where the size parameter D is actually the hydrodynamic radius of diffusing vesicles (Noto et al., 2012).

Exosome morphology was examined *for* scanning electron microscopy analysis. Exosomes isolated and purified from FAPs by TEIR were fixed in 4% (v/v) paraformaldehyde, dehydrated by a series of incubations in 30%, 50%, and 70% (v/v) ethanol and dried on aluminium support for SEM. Exosomes isolated were coated with gold. A SEM LEO 1450VP (Carl Zeiss Meditec, Oberkochen, Germany) was employed to acquire backscattered electron images using 20 keV electrons leading to an information depth of about 1.5 µm. Images with a scan size of 30 × 30 µm were acquired, at a resolution of 1024 × 1024 pixels.

### -Exosome labeling

### Acridine Orange

To detect nucleic acid content, FAPs derived exosomes (10µg) isolated by TEIR, were labeled with acridine orange -AO- (#235474, Sigma) (100 mg/ml) for 30 minutes at room temperature and added to MuSCs in order to reveal by immunofluorescence the RNA exchanged from FAPs to MuSCs.

### PKH-67

To visualize exosomes, FAPs-derived exosomes (10µg) are stained for 5 minutes at room temperature RT with 0.5µl of PKH67 Green Fluorescent Cell Linker Kit for General Cell Membrane Labeling (#P7333, Sigma) and then polished with the exosome spin columns (#4484449, Invitrogen by Thermo Fisher Scientific) following the manufacturer protocol. The stained exosomes are detected by immunofluorescence into myo-fibers and by cyto-fluorimetric analysis in mice muscle after their *in-vivo* injection.

### Exosomal content

### -Protein

Exosomes isolated were lysed for protein extraction in RIPA buffer (50 mM Tris-HCl, pH 7.4; 150 mM NaCl; 1% NP-40; protease inhibitors). The total exosomal protein content was quantified using the micro bicinchoninic acid protein assay (BCA) (#23235, Thermo Fisher scientific).

### Western blot

Western blot was performed using antibodies against the following proteins: rabbit anti-CD63 (1/300, H-193, Santa Cruz Biotechnology), mouse anti-Hsp70 (1/2500, clone BRM-22, Sigma-Aldrich), rabbit anti-Calnexin (1/1000, NB100-1965, Novus Biologicals), rabbit anti-Flotillin-1 (1/200, H-104, Santa Cruz Biotechnology).

As total lysate normalization we used Ponceau quantifiction.

### -microRNA

Total exosomal RNA was extracted with Total Exosome RNA Protein Isolation Kit (#4478545, Thermo Fisher Scientific). TaqMan MicroRNA Assays were performed according to the manufacturer's recommended protocols (Applied Biosystems) The threshold cycle (Ct) were defined as the fractional cycle number at which the fluorescence passes the fixed threshold. U6 snRNA served as an endogenous control for normalization.

In particular, total exosomal-RNA was retro-transcribed using TaqMan MicroRNA Reverse Transcription Kit (#4366596, ThermoFisher Scientific)
For greater sensitivity, the cDNA was pre-amplified using Taqman PREAMP master mix (#4391128, ThermoFisher Scientific) and Megaplex PreAMP Primers and then amplified using high multiplexed Megaplex Primer Pool (#4444766, ThermoFisher Scientific) and the TaqMan 2X Universal Master Mix II (#4440040, ThermoFisher Scientific) on TaqMan rodent MicroRNA A/B cards Array version 3.0 (#4444909, ThermoFisher Scientific).

### qRT-PCR

For microRNA validation, Total exosomal RNA was extracted with Total Exosome RNA Protein Isolation Kit (#4478545, Invitrogen by Thermo Fisher Scientific) and was retro-transcribed using the Qiagen reverse transcription kit (miScript II RT Kit, #218161, Qiagen) and pre-amplified using (miScript PreAMP PCR Kit, # 331451, Qiagen).

Real-time qPCR was performed using (miScript SYBR Green PCR Kit, #218073, Qiagen) and using primers reported in table (KRT). All the conditions are provided in the manufacturer protocol.

### FAPs derived Exosome intra-muscular injection

FAPs-derived exosomes at a final concentration of 10µg in 20µL of PBS1x (0.5µg/µl) were injected 3 times in left Tibialis Anterior (TA) of C57BL6/mdx mice: every 7 days for 21 days. The right TA was injected with vehicle (20 µL PBS1x) following the same timing described for exosomes injection.

### RNA-sequencing

For RNA-sequencing sample preparation, MuSCs and FAPs were freshly isolated by FACS from 6 C57Bl6 mdx male mice 8-weeks old treated or not with TSA for 15 days. RNA was collected using Trizol) reagent (#T9424, Sigma). About 100 ng/µL of total RNA was sent in duplicate to IGA (Istituto di Genomica Applicata, Udine) for RNA sequencing using Illumina TruSeq Stranded Total RNA kit Ribo-Zero GOLD on Illumina Hiseq2500 platform.

### Quantification and statistical analysis

The number of independent experimental replications and precision measures are reported in the figure legends (n, mean ± sem or n, mean ± sd). Statistical analyses were performed using the Microsoft office excel 2016 statistical utilities. Statistical significance was assessed by the two-tailed Student's t test. P-value < 0.05 was considered as statistically significant.

### Data and software availability

The cells positive for the stainings described in the text were quantified using ImageJ software (https://imagej.nih.gov/ij/download.html). The cross-sectional area (CSA), was also calculated using the ImageJ software and Macro seg 5 modif.ijm specific plugin. Fibrotic areas were measured from sections evaluating image analysis algorithms for colour deconvolution. ImageJ was used for image processing, the original image was segmented with three clusters and the plugin assumes images generated by color subtraction (white representing background, blue collagen, and magenta non collagen regions).

FACS profile analysis of MuSCs and FAPs were performed using Flowjo software (https://www.flowjo.com).

The RNA sequencing analysis was performed Mapping more than 20 millions of reads for each sample to the Mus Musculus GRCm38.78 genome using TopHat 2.0.9. Read count was performed with HTSeq-0.6.1p1. Mapped reads were analysed with R-studio using DESeq2 to obtain normalized RPKM, P-Value, P-adjusted and log2fold changes values. Genes were considered differentially expressed if the P-adjusted value was <0.1.

miRNA pathway analysis was performed using miRPath based on predicted miRNA targets provided by the DIANA-microT-CDS algorithm and/or experimentally validated miRNA interactions derived from DIANA-TarBase v6.0 (http://www.microrna.gr/miRPathv2).

### Prediction of miRNA-mRNA interactions and network construction.

Data from RNAseq (padj<0.1) in MuSCs (treated or not with TSA) and Microarray analysis in FAPs (treated or not with TSA) derived exosomes, were uploaded in IPA (https://www.qiagenbioinformatics.com/products/ingenuity-pathway-analysis/). With the IPA tool "miR Target Filter" the miR upregulated in FAPs exosomes were intersected with transcripts down-regulated in MuSCs after TSA treatment. Only interactions experimentally observed and highly predicted were selected for downstream analysis. Notch pathway was extrapolated from IPA database using the tool "grow", while its modulation was predicted by the Molecular Activity Predictor (MAP) tool. The cartoon was realized with IPA path-designer.

### Example 1

### Exosomes mediate FAP's ability to promote MuSe differentiation into multinucleated myotubes

We began to determine the identity of the extracellular mediators of FAP's support to MuSCs in DMD muscles, by performing transwell co-cultures between FAPs isolated from 1.5 month old mdx mice and MuSCs isolated from age-matched mdx mice. In transwell cultures, cells are separated by a membrane of 1 µm pore size that prevents direct cell contact, yet allows reciprocal transfer of soluble mediators of functional interactions between co-cultured cells.

Figure 1 shows that FAPs enhanced the ability of co-cultured MuSCs to differentiate into multinucleated myotubes, as compared to MuSCs cultured alone (Figure 1A and B), as previously reported (Mozzetta et al., 2013).

To determine the relative contribution of extracellular vesicles (EVs (e.g. exosomes) versus non-EV soluble factors, we used the neutral sphingomyelinase inhibitor GW4869, which selectively blocks exosome biogenesis (Kosaka et al., 2010).

Exposure to GW4869 abrogated FAP's ability to enhance MuSCs differentiation into multinucleated myotubes (Figure 1A and B). This evidence indicates that exosomes are essential contributors of FAP-mediated support to MuSCs activity, and implies that FAP-delivered exosomes are transferred to MuSCs, whereby they influence MuSC biological properties.

To analyse the capacity of MuSCs to uptake FAP-derived exosomes, we performed a transwell co-culture in which the potential "source" of exosomes - FAPs - was transfected with the exosomal marker CD63 fused to GFP prior to transwell co-culture with the "acceptor" cells - MuSCs. In this system, detection of GFP into MuSCs reveals the transfer of CD63-labelled exosomes from FAPs to MuSCs. Figure 2 shows that GFP signal was detected in FAPs after transfection with GFP-CD63, but also in MuSCs co-cultured with FAPs previously transfected with GFP-CD63, whereas no GFP signal was detected in MuSCs co-cultured with FAPs previously transfected with a control plasmid (control) (Figure 2A). To conclusively demonstrate the transferring of exosomes from FAPs to MuSCs, we incubated FAPs with the lipidic dye (PKH-67) prior to transwell co-culture with MuSCs. PKH-67 is incorporated into newly generated exosomes, and therefore traces their passage to acceptor cells. PKH-67 staining was invariably detected in FAPs exposed to the dye, and in MuSCs co-cultured with PKH67-labelled FAPs; however, exposure to GW4869 almost completely prevented (more than 80%) the detection of the signal in MuSCs co-cultured with FAPs (Figure 1C, D), indicating that FAP-derived exosomes are uptaken by MuSCs.

We next isolated exosomes from the non-filtered conditioned media (CM) derived from FAPs and analyzed their size distribution by Dynamic Light Scattering (DLS) experiments. To this purpose we have compared two purification procedures, including the commercial available Total Exosome IsolationTM precipitation reagent (TEIR), or the current gold standard isolation method, the differential ultracentrifugations (UC) (Thery et al., 2006; Van Deun et al., 2014). DLS analyses showed for both FAPs-derived vesicles preparations an average hydrodynamic diameter of about 150 nm that is consistent with the standard size of exosomes (Figure 2B) (Raposo and Stoorvogel, 2013). DLS analysis was also complemented with scanning electron microscopy (SEM) analyses of fixed TEIR-isolated exosomes that showed round-shaped FAP-derived exosomes ranging from 100-150 nm in size (Figure 2C). The slight difference in size between SEM and DLS analyses can be explained by the fact that DLS measures the hydrodynamic diameter of native particles in dispersion and is inherently biased towards larger particles (Sokolova, et al., 2011).

Moreover, a western blot analysis of the protein content showed that presumptive exosome markers were abundantly expressed in exosomes TEIR-isolated from FAP's supernatant, including Alix, Hsp70, and to a less extent Flotillin1 and CD63 (Figure 2D). By contrast, exosome preparations was cleared from contaminating cell organelles, as indicated by the absence of Calnexin, an ubiquitously expressed ER protein that was exclusively found in FAP's whole cell fractions (WCL) (Figure 2D).

Collectively, these data demonstrate that functionally active FAPs-derived EVs display typical features of mammalian exosomes (Lötvall et al., 2014; EV-TRACK Consortium, 2017).

### Example 2

### Increased abundance of muscle interstitial exosomes upon regeneration post acute injury, and in young, but not old, dystrophic muscles exposed to HDAC inhibitors

We investigated whether FAP-derived exosomes could be detected during muscle regeneration in physiological versus pathological conditions.

To this purpose, we performed in situ immunofluorescence for CD63, an integral membrane protein enriched in exosomes, on muscle sections from young WT unperturbed (CTR) or regenerating muscles (3 days post injury-cardiotoxin injection, CTX), and from the mouse model of Duchenne Muscular Dystrophy (DMD) - the mdx mice - either 1.5-month young or 1 year old. We also included sections of muscles from mdx mice that were exposed or not to the HDACi Trichostatin A (TSA), which promotes regeneration in young, but not old, mdx mice (Mozzetta et al. 2013; Saccone et al. 2014).

This analysis revealed sporadic CD63 signal in WT unperturbed and old mdx muscles, while a dramatic increase in CD63 signal was observed in regenerating WT and mdx young muscles (Figure 1E, F).

Interestingly, in all experimental conditions the vast majority of CD63 signal was invariably detected in the interstitium between regenerating, embryonal eMyHC-positive fibers, and largely overlapped with interstitial Sca-1 signal, which identifies putative FAP cells.

Notably, muscle of mdx young mice exposed to TSA showed a significant increase of CD63-positive interstitial signal, as compared to young mdx CTR muscles, with levels comparable to those observed in regenerating WT muscles (Figures 1E and F). By contrast, TSA treatment could not increase the CD63 signal in the interstitium of muscles from 1 year-old mdx mice, which have been previously shown to be resistant to the beneficial effects of HDACi (Mozzetta et al. 2013; Saccone et al. 2014). The overlap of interstitial CD63 and Sca1 signals, and the close proximity to eMyHC-positive regenerating myofibers, together with the differential response of young or old mdx muscles to HDACi, suggest that exosomes derived from the expanding population of activated FAPs could be implicated in HDACi-mediated activation of MuSCs to regenerate dystrophic muscles.

### Example 3

### miR-206 mediates the pro-myogenic effects of HDACi-induced exosomes derived from FAPs of dystrophic muscles.

Previous works have implicated microRNA (miRs), as mediators of exosome-regulated biological processes (Valadi et al., 2007; Hunter et al., 2008; Collino et al., 2010; Montecalvo et al., 2012; Vlassov et al., 2012; Boon and Vickers 2013; He et al., 2014; Nakamura et al., 2015; Fry et al. 2017).

To investigate miRs contribution to FAP-derived exosome ability to regulate MuSC activity, we first determined whether FAP-derived exosomes could transfer RNA to MuSCs, by using fluorescent acridine orange (AO) - a specific nucleic acid staining. AO labeled FAPs-derived exosomes were incubated with freshly isolated MuSCs. Confocal microscopy analysis revealed AO-labelled intracellular RNA spots inside recipient MuSCs, within and outside the nuclear membrane (Figure 4A).

We have previously shown that HDACi extensively change the miR expression pattern in FAPs from mdx muscles (Saccone et al., 2014). Therefore, we sought to further investigate the functional impact that blockade miR biogenesis has on muscle regeneration. To this purpose, we evaluated the effect of siRNA-mediated knockdown of Drosha, the RNA-specific endoribonuclease required for microRNA biogenesis, on functional interactions between FAPs isolated from HDACi-treated young mdx mice and MuSCs isolated from untreated young mdx mice. siRNA efficiently down-regulated Drosha expression (by more than 90%) in FAPs (Figure 3C), and drastically reduced the ability of TSA-treated FAPs to enhance MuSC-mediated formation of multinucleated myotubes in transwell co-culture (Figure 3A and B).

We then determined the identity of the miRs within FAP-derived exosomes, by performing a Taqman-based miR expression microarray from exosomes isolated from the supernatant of FAPs derived by mdx mice treated for 15 days with TSA or control vehicle (CTR). This analysis revealed several miRs up-regulated in exosomes from TSA-treated mdx mice.

We annotated the top 14 miRs significantly upregulated in FAPs from TSA-treated mdx mice (Figure 3D and Figure 4B), including miRs that have been previously implicated in muscle processes and development (Greco et al., 2009; Wang, 2013; Zaho et al., 2016; Guess et al., 2015; Nakamura et al., 2016; Yamamoto et al., 2012; Wang et al., 2012). Ingenuity Pathway Analysis (IPA) revealed a number of regulatory pathways potentially affected by these miRs and implicated in the control of MuSCs (Figure 4C). Interestingly, RNAseq data generated from MuSCs isolated from mdx mice treated with TSA versus control vehicle revealed the activation of top pathways (Notch, JAK-STAT and TGFbeta) that were also predicted by IPA analysis of up-regulated miR in exosomes from FAPs of TSA-treated mdx mice (Figure 4D).

Among the TSA-induced exosomal miRs, the most up-regulated was the muscle-specific (myomiR) miR-206 - a central component of skeletal muscle regeneration (Liu et al., 2012; Ma et al., 2015) and HDACi-activated network in dystrophic muscles (Cacchiarelli et al., 2010; Saccone et al., 2014). Of note, transgenic miR-206 upregulation has been exploited as tool for the treatment of DMD and other muscular disorders (Liu et al., 2012; Williams et al., 2009; Fry et al., 2017). Interestingly, in situ hybridization analysis of muscle biopsies from DMD patients at various ages revealed increased levels of interstitial miR-206, as compared to control biopsies (from non DMD boys) (Figure 3E), with a progressive reduction along with the disease progression that coincided with the exhaustion of the regenerative activity and severity of disease development (Figure 3F and 3G). The same pattern could be observed in mdx mice, in which an abundant miR-206 signal was detected in the interstitium of tibialis anterior muscles, overlapping with the immunofluorescence staining of the FAP's marker Sca-1 (Figure 3H). Of note, treatment of young mdx mice (1.5 months of age) with TSA, which promotes regeneration and inhibits fibrosis (Mozzetta et al., 2013; Saccone et al., 2014), increased the amount of interstitial miR-206 signal associated with Sca-1 staining (Figure 3H). We performed a quantitative assessment of exosomal miR-206, by isolating whole tibialis anterior (TA) muscles from TSA-treated mdx muscle or untreated control mdx mice, followed by gentle dissociation and exosome isolation. This procedure enriches for interstitial material, as it minimizes the exosome content in myofibers. qPCR showed a ~7 fold increase of miR-206 in TSA-treated mdx muscle as compared to untreated control (Figure 3I).

We then evaluated the impact of miR-206 on FAP-derived exosome ability to enhance MuSC-mediated formation of multinucleated myofibers. To this purpose, we purified exosomes from FAPs isolated from TSA-treated 1.5 month-old mdx mice and immediately transfected with antagomiR-206 (TSA-A206) or vehicle (TSA), and tested their ability to promote MuSC-mediated formation of multinucleated myotubes. Exosomes purified from FAPs transfected with antagomiR-206 showed a reduced ability to promote MuSC-mediated formation of multinucleated myotubes, as compared to exosomes purified from FAPs isolated from vehicle (TSA), as well as to FAPs from untreated (CTR) mdx mice (Figure 5A-C). Thus, miR-206 is a main component of the FAP-derived exosomes that support MuSC differentiation ability ex vivo.

### Example 4

### MiR-206 in exosomes isolated from FAPs of HDACi-treated dystrophic muscles is required to promote MuSCs activation and expansion.

To gain mechanistic insights into the pro-myogenic activity of exosomes derived from FAPs of HDACi-treated dystrophic muscles, we searched for putative target genes of miR-206 that matched with downregulated genes in our RNAseq datasets obtained from MuSCs of TSA-treated mdx mice (Figure 6A). A downstream analysis was carried only for miR-206 targets that have been already annotated or predicted with high confidence. Ingenuity pathway analysis (IPA) revealed a potential effect of miR-206 on various signaling pathways involved into muscle development and disease, including utrophin (UTRN), Pax7 and Notch3 (Rosenberg et al., 2006; Amirouche et al., 2014; Cacchiarelli et al., 2010; Chen et al., 2010; Gagan et al., 2012) (Figure 6A).

We decided to focus on miR-206 regulation of Notch3, as negative regulator of MuSC differentiation, because previous works demonstrated that miR-206 down-regulation of Notch3 promotes MuSC differentiation (Gagan et al., 2012). While both Notch3 and Notch1 transcripts were reduced in MuSCs isolated from mdx mice treated with TSA, we observed a selective downregulation of Notch3 transcripts in MuSCs isolated from untreated mdx mice and cultured with exosomes derived from FAPs of TSA-treated mdx mice (Figure 5D and 5E). In keeping with the opposite roles of Notch members in MuSCs activation (Kitamoto and Hanaoka, 2010), we reasoned that selective downregulation of Notch3 by FAP-derived exosomal miR-206 could promote MuSC activation and self-renewal. To investigate this hypothesis, we evaluated the effect of FAP-derived exosomes from HDACi-treated or control-treated mdx mice on MuSCs within single fibres isolated from WT mice. Freshly isolated single muscle fibres from tibialis anterior, extensor digitorum longus, gastrocnemius and soleus of C57BL6J mice were incubated with FAP-derived exosomes or whole conditioned media, and their effect on MuSCs was evaluated by monitoring the expression of Pax7, MyoD and DNA synthesis (EdU incorporation). In this experimental setting, the uptake of FAP-derived exosomes by MuSCs from single fibers was first confirmed by staining of lipidic dye PKH-67 used on purified vesicles (Figure 6B).

Figures 5 F and G show that the whole supernatant derived from FAPs of control-treated mdx mice increased the number of Pax7+/MyoD+ MuSCs that exhibited enhanced DNA synthesis (Edu+) and could be therefore considered activated MuSCs (Zammit et al., 2004). Moreover, the whole supernatant derived from FAPs of TSA-treated mdx mice could further augment this effect. While a drastic reduction in the number of quiescent (Pax7+ MyoD-) and differentiation-committed (Pax7- MyoD+) MuSCs was observed upon incubation with the whole supernatant of FAPs isolated from control-treated mdx mice and pre-exposed to GW4869, the number of Pax7+/MyoD+ MuSCs with high DNA synthesis (Edu+) was not altered in these conditions (Figures 5F and G). By contrast, GW4869 treatment of FAPs from TSA-treated mdx mice drastically reduced the ability of the whole supernatant to increase all MuSC populations as well as their proliferation (Figures 5F and G).

This result indicates that the ability of FAPs from mdx mice to support MuSCs activation and differentiation, but not expansion of Pax7+/MyoD+ MuSCs, relies on exosomal factors, while the ability of FAPs from HDACi-treated mdx mice to promote expansion of MuSCs entirely depends on exosomes. Indeed, exosomes from FAPs of TSA-treated (but not from control-treated) mdx mice increased the number of Pax7+/MyoD+ MuSCs (Figure 5F and G).

We then investigated the relative contribution of exosomal miR-206 in mediating the ability of FAPs from TSA-treated mdx mice to support MuSC activation, expansion and differentiation, by transfecting miR-206 antagomiR in FAPs 24 hours prior to the purification of the exosomes. This blockade of miR-206 could completely abrogate FAP ability to support MuSCs, pointing to a central role of exosomal miR-206 in mediating HDACi ability to promote functional interactions between dystrophic FAPs and MuSCs (Figure 5F and G).

### Example 5

### Exosomal miR-206 is required to promote compensatory regeneration and reduce fibrosis, but not inflammation, in dystrophic muscles.

We finally investigated whether FAP-derived exosomes (Exo-FAPs) could promote muscle regeneration in vivo, within the context of the mouse model of DMD - the mdx mice - in which HDACi exert beneficial effects, by promoting compensatory regeneration, and by reducing fibrosis and inflammation (Minetti et al. 2006). As we sought to evaluate to what extent these effects are accounted by FAP-derived exosomes, we compared the ability to stimulate regeneration, reduce fibrosis and inflammation in mdx muscles by systemic exposure to TSA (or vehicle control), as administered via daily intra-peritoneal injection, versus intramuscular injection of exosomes purified from FAPs isolated from mdx mice that were previously treated with TSA (Exo-FAPs TSA) or vehicle (Exo-FAPs CTR). The exosome injection was repeated every 7 days for 21 days, a timeframe that we found compatible to quantify the histological parameters of productive regeneration, fibrosis and inflammation, and is equivalent to the time of systemic exposure to TSA. We first verified the successful injection of Exo-FAPs, with PKH-67 staining, by flow cytometry. For this assay, we exploited the ability of CytoFLEX to detect nano-molecules. Using a mixture of non-fluorescent silica beads and fluorescent (green) latex beads with sizes ranging from 110 nm to 1300 nm, we revealed PKH-67 staining of about 67% of exosomes, prior to their injection (Figure 7A). Using this procedure we could verify the persistence of injected exosomes, by detection of the green signal in transplanted muscles (Figure 7B).

Exo-FAPs TSA injected muscles showed an increase in both fibers caliber, as measured by cross sectional area (CSA), and embryonic-MyHC-positive myofibers (eMyHC), that were comparable to those produced by systemic delivery of TSA (Figure 8A and B). Likewise, we observed a comparable ability of Exo-FAPs TSA intramuscular transplantation and systemic delivery of TSA to increase muscle fiber area fraction (MFAF), reduce fibrosis (Figure 8C and D) and inflammation (Figure 8E) in mdx muscles. A significant reduction of MPO-positive area similar to what calculated for TSA intra-peritoneal injected mice, was observed in Tibialis anterior muscles of all animals (Figure 8E).

To evaluate the contribution of miR-206 to the effects of FAP-derived exosomes on mdx mice, FAPs isolated from TSA-treated mice were transfected with AntagomiR-206 (Exo FAPs TSA A-206) prior to exosome isolation and injection into Tibialis Anterior of mdx mice. While exosomes isolated from FAPs+A206 showed a reduced ability to stimulate muscle regeneration (Figure 8A and B) and reduce fibrosis (Figure 8C and D), they could still decrease inflammation, indicating a selective contribution of miR-206 to promote regeneration and reduce fibrosis. By contrast, the anti-inflammatory effects of FAP-derived exosomes appear to be independent on miR-206 (Figure 8E).

### Example 6

### FAPs isolated from dystrophic muscles can be manipulated in vitro using HDACi in order to obtain exosomes able to promote MuSCs differentiation

HDACi extensively change the miR expression pattern in FAPs derived exosomes isolated from mdx muscles, even if the treatment is performed *in vitro* with a result comparable with that observed into mdx treated *in vivo* with HDACi (Figure 9A).

To evaluate the biological properties of these exosomes, we purified them from FAPs isolated from 1.5 month-old mdx mice, cultured and exposed to TSA 15 hours before refreshing the cell with cell free medium, and tested their ability to promote MuSC-mediated formation of multinucleated myotubes.

Exosomes purified from FAPs in vitro treated with TSA (Exo-TSA in vitro) showed an enhanced ability to promote MuSC-mediated formation of multinucleated myotubes as compared to exosomes purified from FAPs isolated from untreated (Exo-CTR) mdx mice (Figure 9B-C).

### REFERENCES

Amirouche A, Tadesse H, Miura P, Bélanger G, Lunde JA, Côté J, Jasmin BJ. (2014). Converging pathways involving microRNA-206 and the RNA-binding protein KSRP control post-transcriptionally utrophin A expression in skeletal muscle. Nucleic Acids Res. 42(6):3982-3997.
Bentzinger CF, Wang YX, Dumont NA, Rudnicki MA. (2013). Cellular dynamics in the muscle satellite cell niche. EMBO Rep. 14(12):1062-1072.
Bettica P, Petrini S, D'Oria V, D'Amico A, Catteruccia M, Pane M, Sivo S, Magri F, Brajkovic S, Messina S, et al. (2016). Histological effects of givinostat in boys with Duchenne muscular dystrophy. Neuromuscul Disord. 26(10):643-649.
Blau HM, Cosgrove BD, Ho AT. (2015). The central role of muscle stem cells in regenerative failure with aging. Nat Med. 21(8):854-862.
Boon RA, Vickers KC. (2013). Intercellular transport of microRNAs. Arterioscler Thromb Vasc Biol.; 33(2):186-192
Braicu C, Tomuleasa C, Monroig P, Cucuianu A, Berindan-Neagoe I, Calin GA. (2015). Exosomes as divine messengers: are they the Hermes of modern molecular oncology? Cell Death Differ. 22(1):34-45.
Cacchiarelli D, Martone J, Girardi E, Cesana M, Incitti T, Morlando M, Nicoletti C, Santini T, Sthandier O, Barberi L, et al. (2010). MicroRNAs Involved in Molecular Circuitries Relevant for the Duchenne Muscular Dystrophy Pathogenesis Are Controlled by the Dystrophin/nNOS Pathway. Cell Metab. 12:341-351.
Carpentieri A, Cozzoli E, Scimeca M, Bonanno E, Sardanelli AM, Gambacurta A. (2016). Differentiation of human neuroblastoma cells toward the osteogenic lineage by mTOR inhibitor. Cell Death Dis. 21;7:e2202.
Chang NC, Chevalier FP, Rudnicki MA. (2016) Satellite Cells in Muscular Dystrophy - Lost in Polarity. Trends Mol Med. 22(6):479-496.
Chen JF, Tao Y, Li J, Deng Z, Yan Z, Xiao X, Wang DZ. (2010). microRNA-1 and microRNA-206 regulate skeletal muscle satellite cell proliferation and differentiation by repressing Pax7. J Cell Biol. 190(5):867-79.
Choi JS, Yoon HI, Lee KS, Choi YC, Yang SH, Kim IS, Cho YW. (2016). Exosomes from differentiating human skeletal muscle cells trigger myogenesis of stem cells and provide biochemical cues for skeletal muscle regeneration. Journal of Controlled Release. 222:107- 115
Collino F, Deregibus MC, Bruno S, Grange C, Tetta C, Camussi G. (2010). Microvesicles derived from adult human bone marrow and tissue specific mesenchymal stem cells shuttle selected pattern of miRNAs. PLoS ONE. 5(7):e11803.
Colussi C., Mozzetta C., Gurtner A., Illi B., Straino S., Ragone G., Pescatori M., Zaccagnini G., Rosati G., Minetti G., et al. (2008). A Common Epigenetic Mechanism Underlies Nitric Oxide Donors and Histone Deacetylase Inhibitors Effect in Duchenne Muscular Dystrophy. Proc. Natl. Acad. Sci 105: 19183-19187.
Consalvi S, Saccone V, Giordani L, Minetti G, Mozzetta C, Puri P.L. (2011) Histone deacetylase inhibitors in the treatment of muscular dystrophies: epigenetic drugs for genetic diseases. Mol Med. 17(5-6):457-465.
Consalvi S, Mozzetta C, Bettica P, Germani M, Fiorentini F, Del Bene F, Rocchetti M, Leoni F, Mascagni P, Puri P.L, Saccone V. (2013). Preclinical studies in the mdx mouse model of Duchenne Muscular Dystrophy with the Histone Deacetylase inhibitor Givinostat. Mol Med. 19:79-87.
Dumont NA, Wang YX, Rudnicki MA. (2015a). Intrinsic and extrinsic mechanisms regulating satellite cell function. Development 142(9):1572-1581.
Dumont NA, Wang YX, von Maltzahn J, Pasut A, Bentzinger CF, Brun CE, Rudnicki MA. (2015b). Dystrophin expression in muscle stem cells regulates their polarity and asymmetric division. Nat Med. 21(12):1455-1463.
EV-TRACK Consortium, Deun J V, Mestdagh P, Agostinis P, Akay Ö, Anand S, Anckaert J, Martinez Z A, Baetens T, Beghein E, et al. (2014). EV-TRACK: transparent reporting and centralizing knowledge in extracellular vesicle research. Nature Methods. 14:228-232
Farup J, Madaro L, Puri PL, Mikkelsen UR. (2015). Interactions between muscle stem cells, mesenchymal-derived cells and immune cells in muscle homeostasis, regeneration and disease. Cell Death and Disease. 6:e1830
Fry CS, Kirby TJ, Kosmac K, McCarthy JJ, Peterson CA. (2017). Myogenic Progenitor Cells Control Extracellular Matrix Production by Fibroblasts during Skeletal Muscle Hypertrophy. Cell Stem Cell. 20(1):56-69.
Gagan J, Dey BK, Layer R, Yan Z, Dutta A. (2012). Notch3 and Mef2c proteins are mutually antagonistic via Mkp1 protein and miR-1/206 microRNAs in differentiating myoblasts. J Biol Chem. 287(48):40360-40370.
Greco S, De Simone M, Colussi C, Zaccagnini G, Fasanaro P, Pescatori M, Cardani R, Perbellini R, Isaia E, Sale P, et al. (2009). Common micro-RNA signature in skeletal muscle damage and regeneration induced by Duchenne muscular dystrophy and acute ischemia. FASEB J. 23(10):3335-3346.
Guess MG, Barthel KK, et al. (2015). miR-30 family microRNAs regulate myogenic differentiation and provide negative feedback on the microRNA pathway. PLoS One. 10(2):e0118229.
He WA, Calore F, Londhe P, Canella A, Guttridge DC, Croce CM. (2014). Microvesicles containing miRNAs promote muscle cell death in cancer cachexia via TLR7. Proc Natl Acad Sci USA. 111(12):4525-4529.
Heredia JE, Mukundan L, Chen FM, Mueller AA, Deo RC, Locksley RM, Rando TA, Chawla A. (2013). Type 2 innate signals stimulate fibro/adipogenic progenitors to facilitate muscle regeneration. Cell 153(2): 376-388.
Hoffman, E. P., and Dressman, D. (2001). Molecular pathophysiology and targeted therapeutics for muscular dystrophy. Trends Pharmacol Sci 22, 465-470.
Hunter MP, Ismail N, Zhang X, Aguda BD, Lee EJ, Yu L, Xiao T, Schafer J, Lee ML, Schmittgen TD, et al. (2008). Detection of miR expression in human peripheral blood microvesicles. PLoS ONE. 3(11):e3694.
Iraci N, Leonardi T, Gessler F, Vega B, Pluchino S. (2016). Focus on Extracellular Vesicles: Physiological Role and Signalling Properties of Extracellular Membrane Vesicles. Int J Mol Sci. 17(2):171.
Joe AW, Yi L, Natarajan A, Le Grand F, So L, Wang J, Rudnicki MA, Rossi FM (2010). Muscle injury activates resident fibro/adipogenic progenitors that facilitate myogenesis. Nat Cell Biol. 2:153-163
Kharraz Y, Guerra J, Mann CJ, Serrano AL, Muñoz-Cánoves P. (2013). Macrophage plasticity and the role of inflammation in skeletal muscle repair. Mediators of Inflammation. ID:491497.
Kitamoto T, Hanaoka K. (2010) Notch3 null mutation in mice causes muscle hyperplasia by repetitive muscle regeneration. Stem Cells. 28(12):2205-2216.
Kosaka N, Iguchi H, Yoshioka Y, Takeshita F, Matsuki Y, Ochiya T. (2010). Secretory mechanisms and intercellular transfer of microRNAs in living cells. J Biol Chem. 285(23):17442-52.
Lee Y, El Andaloussi S, Wood MJ. (2012). Exosomes and microvesicles: extracellular vesicles for genetic information transfer and gene therapy. 21(R1):R125-134.
Lemos DR, Babaeijandaghi F, Low M, Chang CK, Lee ST, Fiore D, Zhang RH, Natarajan A, Nedospasov SA, Rossi FM. (2015). Nilotinib reduces muscle fibrosis in chronic muscle injury by promoting TNF-mediated apoptosis of fibro/adipogenic progenitors. Nat Med. 21(7):786-794.
Liu N, Williams AH, Maxeiner JM, Bezprozvannaya S, Shelton JM, Richardson JA, Bassel-Duby R, Olson EN. (2012). microRNA-206 promotes skeletal muscle regeneration and delays progression of Duchenne muscular dystrophy in mice. J Clin Invest. 122(6):2054-2065.
Lötvall J, Hill AF, Hochberg F, Buzás EI, Di Vizio D, Gardiner C, Gho YS, Kurochkin IV, Mathivanan S, Quesenberry P, et al. (2014). Minimal experimental requirements for definition of extracellular vesicles and their functions: a position statement from the International Society for Extracellular Vesicles Journal of Extracellular Vesicles. 3:26913
Ma G, Wang Y, Li Y, Cui L, Zhao Y, Zhao B, Li K. (2015). MiR-206, a key modulator of skeletal muscle development and disease. Int J Biol Sci. 11(3):345-352.
Mauro A. Satellite cell of skeletal muscle fibers. (1961). J Biophys Biochem Cytol. 9:493-495.
Minetti G. C., Colussi c., Adami R., Serra C., Mozzetta C., Parente V., Illi B., Fortuni S., Straino S., Gallinari P., et al. (2006). Functional and morphological recovery of dystrophic muscles in mice treated with deacetylase inhibitors. Nature Medicine 12(10):1147-1150.
Montecalvo A, Larregina AT, Shufesky WJ, Stolz DB, Sullivan ML, Karlsson JM, Baty CJ, Gibson GA, Erdos G, Wang Z, et al. (2012). Mechanism of transfer of functional microRNAs between mouse dendritic cells via exosomes. Blood. 119(3):756-766.
Moyer AL, Wagner KR. (2011). Regeneration versus fibrosis in skeletal muscle. Curr Opin Rheumatol. 23(6):568-573.
Moyle LA, Zammit PS. (2014) Isolation, culture and immunostaining of skeletal muscle fibres to study myogenic progression in satellite cells Methods Mol Biol. 1210:63-78.
Mozzetta C, Consalvi S, Saccone V, Tierney M, Diamantini A, Mitchell KJ, Marazzi G, Borsellino G, Battistini L, Sassoon D, et al. (2013 ). Fibroadipogenic progenitors mediate the ability of HDAC inhibitors to promote regeneration in dystrophic muscles of young, but not old Mdx mice. EMBO molecular medicine. 5(4): 626-663.
Nakamura Y, Miyaki S, Ishitobi H, Matsuyama S, Nakasa T, Kamei N, Akimoto T, Higashi Y, Ochi M. (2015). Mesenchymal-stem-cell-derived exosomes accelerate skeletal muscle regeneration. FEBS Lett. 589(11):1257-1265.
Noto R, Santangelo MG, Ricagno S, Mangione MR, Levantino M, Pezzullo M, Martorana V, Cupane A, Bolognesi M, Manno M. (2012). The tempered polymerization of human neuroserpin. PLoS One. 7:e32444).
Pasut A, Jones AE, Rudnicki MA. (2013) Isolation and culture of individual myofibers and their satellite cells from adult skeletal muscle. J Vis Exp. 73:e50074
Pena JTG., Sohn-Lee C., Rouhanifard SH. Ludwig J., Hafner M., Mihailovic, A. Lim, C. Holoch, D. Berninger, P. Zavolan M., and Tuschl T. (2009). MiRNA in situ hybridization in mammalian tissues fixed with formaldehyde and EDC. Nat Methods. 6(2): 139-141.
Raposo G, Stoorvogel W. (2013). Extracellular vesicles: exosomes, microvesicles, and friends. J. Cell Biol. 4:373-383.
Rosenberg MI, Georges SA, Asawachaicharn A, Analau E, Tapscott SJ. (2006). MyoD inhibits Fstl1 and Utrn expression by inducing transcription of miR-206. J Cell Biol. 175(1):77-85.
Ruegg UT (2013). Pharmacological prospects in the treatment of Duchenne muscular dystrophy. Curr Opin Neurol. 5:577-584.
Saccone V, Consalvi S, Giordani L, Mozzetta C, Barozzi I, Sandona M, Ryan T, Rojas-Muñoz A, Madaro L, Fasanaro P, et al. (2014). HDAC-regulated myomiRs control BAF60 variant exchange and direct the functional phenotype of fibro-adipogenic progenitors in dystrophic muscles. Genes Dev. 28(8): 841-857.
Sokolova V, Ludwig AK, Hornung S, Rotan O, Horn PA, Epple M, Giebel B. (2011). Characterisation of exosomes derived from human cells by nanoparticle tracking analysis and scanning electron microscopy. Colloids Surf B Biointerfaces. 87:146-150
Théry C, Amigorena S, Raposo G, Clayton A. (2006). Isolation and characterization of exosomes from cell culture supernatants and biological fluids. Curr Protoc Cell Biol.; Chapter 3:Unit 3.22.
Tidball JG. (2011). Mechanisms of muscle injury, repair, and regeneration. Compr Physiol. 1(4):2029-2062.
Tidball JG, Dorshkind K, Wehling-Henricks M. (2014). Shared signaling systems in myeloid cell-mediated muscle regeneration. Development. 141(6):1184-1196.
Tucciarone L. Etxaniz U, Sandoná M, Consalvi S, Puri PL, Saccone V. (2018). Advanced Methods to Study the Cross Talk Between Fibro-Adipogenic Progenitors and Muscle Stem Cells. Methods Mol Biol. 1687:231-256.
Uezumi A, Fukada S, Yamamoto N, Takeda S, Tsuchida K. (2010) Mesenchymal progenitors distinct from satellite cells contribute to ectopic fat cell formation in skeletal muscle. Nat Cell Biol. 2:143-152.
Uezumi A, Ito T, Morikawa D, Shimizu N, Yoneda T, Segawa M, Yamaguchi M, Ogawa R, Matev MM, Miyagoe-Suzuki Y, et al. (2011) Fibrosis and adipogenesis originate from a common mesenchymal progenitor in skeletal muscle. J Cell Sci. 124:3654-3664.
Valadi H, Ekstrom K, Bossios A, Sjostrand M, Lee JJ, Lotvall JO. (2007) Exosome-mediated transfer of mRNAs and microRNAs is a novel mechanism of genetic exchange between cells. Nat Cell Biol.. 9(6):654-659.
Van Deun J, Mestdagh P, Sormunen R, Cocquyt V, Vermaelen K, Vandesompele J, Bracke M, De Wever O, Hendrix A. (2014). The impact of disparate isolation methods for extracellular vesicles on downstream RNA profiling. J Extracell Vesicles. 3:24858.
Vlassov AV, Magdaleno S, Setterquist R, Conrad R. Exosomes: current knowledge of their composition, biological functions, and diagnostic and therapeutic potentials. (2012) Biochim Biophys Acta. 1820(7):940-948.
Wang L, Zhou L, Jiang P, Lu L, Chen X, Lan H, Guttridge DC, Sun H, Wang H. (2012). Loss of miR-29 in myoblasts contributes to dystrophic muscle pathogenesis. Mol Ther. 20(6):1222-1233.
Wang XH. (2013). MicroRNA in myogenesis and muscle atrophy. Curr Opin Clin Nutr Metab Care. 16(3):258-266.
Williams AH, Valdez G, Moresi V, Qi X, McAnally J, Elliott JL, Bassel-Duby R, Sanes JR, Olson EN. (2009). MicroRNA-206 delays ALS progression and promotes regeneration of neuromuscular synapses in mice. Science. 326(5959):1549-1554.
Yamamoto H, Morino K, Nishio Y, Ugi S, Yoshizaki T, Kashiwagi A, Maegawa H. (2012). MicroRNA-494 regulates mitochondrial biogenesis in skeletal muscle through mitochondrial transcription factor A and Forkhead box j3. Am J Physiol Endocrinol Metab. 303(12):E1419-E1427.
Zammit PS, Golding JP, Nagata Y, Hudon V, Partridge TA, Beauchamp JR. (2004). Muscle satellite cells adopt divergent fates: a mechanism for self-renewal? J Cell Biol. 166(3):347-357.
Zhao Q, Kang Y, Wang HY, Guan WJ, Li XC, Jiang L, He XH, Pu YB, Han JL, Ma YH, et al. (2016) Expression profiling and functional characterization of miR-192 throughout sheep skeletal muscle development. Sci Rep. 6:30281.

## Claims

1. An isolated extracellular vesicle obtained from fibro-adipogenic progenitors (FAPs) cells previously exposed to histone deacetylase (HDAC) inhibitor, wherein said histone deacetylase inhibitor is selected from the group consisting of Trichostatin A, MS-275, suberanilohydroxamic acid (SAHA) and Givinostat, for use in the treatment of a muscular dystrophy.

2. The vesicle for the use according to claim 1, which is an exosome.

3. The vesicle for the use according to claim 1 or 2, wherein said fibro-adipogenic progenitor cells originate from the dystrophic muscle of a subject.

4. The vesicle for the use according to anyone of claims 1-3 wherein said vesicle is obtained by a method including a) providing a population of fibro-adipogenic progenitor (FAP) cells which have been exposed to HDAC inhibitors and b) isolating a plurality of exosomes from said cells.

5. The vesicle for the use according to anyone of claims 1-4, wherein said fibro-adipogenic progenitors (FAPs) are exposed to one or more histone deacetylase (HDAC) inhibitors by their treatment *in vitro* with said HDAC inhibitors.

6. The vesicle for the use according to claim 5, wherein said vesicle is obtained by a method including a) providing a population of fibro-adipogenic progenitors (FAP) cells, preferably isolated from a dystrophic muscle, b) exposing said cells *in vitro* to HDAC inhibitors and c) isolating a plurality of exosomes from said cells.

7. The vesicle for the use according to anyone of claims 1-4, wherein said fibro-adipogenic progenitors (FAPs) are previously exposed to one or more histone deacetylase (HDAC) inhibitors by previous treatment of a dystrophic subject, animal or human, with said HDAC inhibitor and subsequent isolation of said FAPs from the dystrophic muscle of said subject.

8. The vesicle for the use according to claim 7, wherein said vesicle is obtained by a method including a) providing a population of fibro-adipogenic progenitor (FAP) cells isolated from a dystrophic muscle of a subject, said subject being previously treated with HDAC inhibitors, and b) isolating a plurality of exosomes from said cells.

9. The vesicle for the use according to anyone of claims 1-8 wherein said vesicle comprises at least one microRNA selected from the group consisting of: miR-206-3p, miR-542-5p, miR-449a-5p, miR-342-3p, miR-320-3p, miR-192-5p, miR-423-5p, miR-376a-3p, miR-145a-5p, miR-224-5p, miR-30a-5p, miR-494-3p, miR-29a-3p and miR-7b-5p.

10. The vesicle for the use according to claim 9, which is enriched in at least one of the miRNAs listed in claim 9.

11. The vesicle for the use according to claim 9 wherein it comprises the following microRNAs: miR-206-3p, miR-542-5p, miR-449a-5p, miR-342-3p, miR-320-3p, miR-192-5p, miR-423-5p, miR-376a-3p, miR-145a-5p, miR-224-5p, miR-30a-5p, miR-494-3p, miR-29a-3p and miR-7b-5p.

12. A composition comprising a plurality of vesicles of anyone of claims 1-11 for use in the treatment of a muscular dystrophy.

13. The vesicle for the use according to anyone of claims 1-11 or the composition for use according to claim 12 wherein said muscular dystrophy is selected from the group consisting of: Duchenne Muscular Dystrophy, Becker Muscular Dystrophy, Limb-Girdle Muscular Dystrophy and other forms of muscular dystrophies and/or muscle disorders sharing common pathogenesis with said forms.

14. The vesicle for the use according to anyone of claims 1-11 or the composition for use according to claim 12 wherein said muscular dystrophy is the Duchenne Muscular Dystrophy (DMD).

15. The vesicle for the use according to claim 14, wherein said vesicle is administered to a subject which is in an advanced phase of the disease.

16. A pharmaceutical composition containing at least one vesicle according to anyone of claims 1-11 together with at least one pharmaceutically acceptable vehicle and/or excipient for use in the treatment of a muscular dystrophy, in particular in the treatment of Duchenne Muscular Dystrophy.

## Patentansprüche

1. Isoliertes extrazelluläres Vesikel, das aus fibro-adipogenen Vorläuferzellen (FAPs) gewonnen wird, die zuvor einem Histon-Deacetylase-Inhibitor (HDAC) ausgesetzt wurden, wobei der Histon-Deacetylase-Inhibitor aus der Gruppe ausgewählt ist, die aus Trichostatin A, MS-275, Suberanilohydroxamsäure (SAHA) und Givinostat gebildet ist, zur Verwendung bei der Behandlung einer Muskeldystrophie.

2. Vesikel für die Verwendung nach Anspruch 1, das ein Exosom ist.

3. Vesikel zur Verwendung nach Anspruch 1 oder 2, wobei die fibro-adipogenen Vorläuferzellen aus dem dystrophischen Muskel eines Patienten stammen.

4. Vesikel zur Verwendung nach einem der Ansprüche 1-3, wobei das Vesikel durch ein Verfahren erhalten wird, das a) das Bereitstellen einer Population von fibro-adipogenen Vorläuferzellen (FAP), die HDAC-Inhibitoren ausgesetzt wurden, und b) das Isolieren einer Vielzahl von Exosomen aus den Zellen umfasst.

5. Vesikel zur Verwendung nach einem der Ansprüche 1-4, wobei die fibro-adipogenen Vorläufer (FAPs) durch deren in vitro Behandlung mit den HDAC-Inhibitoren einem oder mehreren Histon-Deacetylase (HDAC)-Inhibitoren ausgesetzt werden.

6. Vesikel zur Verwendung nach Anspruch 5, wobei das Vesikel durch ein Verfahren erhalten wird, das a) das Bereitstellen einer Population von fibro-adipogenen Vorläuferzellen (FAP), vorzugsweise isoliert aus einem dystrophischen Muskel isoliert wurden, und b) das Aussetzen der Zellen in vitro gegenüber HDAC-Inhibitoren und c) das Isolieren einer Vielzahl von Exosomen aus den Zellen umfasst.

7. Vesikel zur Verwendung nach einem der Ansprüche 1 bis 4, wobei die fibro-adipogenen Vorläufer (FAPs) zuvor einem oder mehreren Histon-Deacetylase (HDAC)-Inhibitoren durch eine vorherige Behandlung eines dystrophischen Patienten, Mensch oder Tier, mit dem HDAC-Inhibitoren und anschließender Isolierung der FAPs aus dem dystrophischen Muskel des Patienten ausgesetzt wurden.

8. Vesikel zur Verwendung nach Anspruch 7, wobei das Vesikel durch ein Verfahren erhalten wird, das Folgendes umfasst: a) Bereitstellen einer Population von fibro-adipogenen Vorläuferzellen (FAP), die aus einem dystrophischen Muskel eines Patienten isoliert wurden, wobei der Patient zuvor mit HDAC-Inhibitoren behandelt wurde, und b) Isolieren einer Vielzahl von Exosomen aus diesen Zellen.

9. Vesikel zur Verwendung nach einem der Ansprüche 1-8, wobei das Vesikel zumindest eine microRNA umfasst, die aus der Gruppe ausgewählt ist, die aus miR-206-3p, miR-542-5p, miR-449a-5p, miR-342-3p, miR-320-3p, miR-192-5p, miR-423-5p, miR-376a-3p, miR-145a-5p, miR-224-5p, miR-30a-5p, miR-494-3p, miR-29a-3p und miR-7b-5p gebildet ist.

10. Vesikel zur Verwendung nach Anspruch 9, das mit zumindest einer der in Anspruch 9 aufgeführten miRNAs angereichert ist.

11. Vesikel zur Verwendung nach Anspruch 9, wobei dieses die folgenden microRNAs umfasst: miR-206-3p, miR-542-5p, miR-449a-5p, miR-342-3p, miR-320-3p, miR-192- 5p, miR-423-5p, miR-376a-3p, miR-145a-5p, miR-224-5p, miR-30a-5p, miR-494-3p, miR-29a-3p und miR-7b-5p.

12. Zusammensetzung, die eine Vielzahl von Vesikeln nach einem der Ansprüche 1-11 zur Verwendung bei der Behandlung einer Muskeldystrophie umfasst.

13. Vesikel zur Verwendung nach einem der Ansprüche 1-11 oder die Zusammensetzung zur Verwendung nach Anspruch 12, wobei die Muskeldystrophie aus der Gruppe ausgewählt ist, die aus einer Duchenne-Muskeldystrophie, Becker-Muskeldystrophie, Extremitätengürtel-Muskeldystrophie und anderen Formen von Muskeldystrophien und/oder Muskelerkrankungen gebildet ist, die eine gemeinsame Pathogenese mit den genannten Formen teilen.

14. Vesikel zur Verwendung nach einem der Ansprüche 1-11 oder die Zusammensetzung zur Verwendung nach Anspruch 12, wobei die Muskeldystrophie die Duchenne-Muskeldystrophie (DMD) ist.

15. Vesikel für die Verwendung nach Anspruch 14, wobei das Vesikel einem Patienten verabreicht wird, der sich in einer fortgeschrittenen Phase der Krankheit befindet.

16. Pharmazeutische Zusammensetzung, die zumindest ein Vesikel nach einem der Ansprüche 1-11 zusammen mit zumindest einem pharmazeutisch akzeptablen Träger und/oder Hilfsstoff zur Verwendung bei der Behandlung einer Muskeldystrophie, insbesondere bei der Behandlung der Duchenne-Muskeldystrophie, enthält.

## Revendications

1. Vésicule extracellulaire isolée obtenue à partir de cellules progénitrices fibro-adipogènes (FAP) préalablement exposées à un inhibiteur d'histone désacétylase (HDAC), où ledit inhibiteur d'histone désacétylase est choisi dans le groupe consistant en la trichostatine A, MS-275, l'acide subéranilohydroxamique (SAHA) et le Givinostat, destinée à être utilisée dans le traitement d'une dystrophie musculaire.

2. Vésicule destinée à être utilisée selon la revendication 1, qui est un exosome.

3. Vésicule destinée à être utilisée selon la revendication 1 ou 2, où lesdites cellules progénitrices fibro-adipogènes proviennent du muscle dystrophique d'un sujet.

4. Vésicule destinée à être utilisée selon l'une quelconque des revendications 1 à 3, où ladite vésicule est obtenue par un procédé incluant a) la fourniture d'une population de cellules progénitrices fibro-adipogènes (FAP) qui ont été exposées à des inhibiteurs de HDAC et b) l'isolement d'une pluralité d'exosomes à partir desdites cellules.

5. Vésicule destinée à être utilisée selon l'une quelconque des revendications 1 à 4, où lesdits progéniteurs fibro-adipogènes (FAP) sont exposés à un ou plusieurs inhibiteurs d'histone désacétylase (HDAC) par leur traitement *in vitro* avec lesdits inhibiteurs de HDAC.

6. Vésicule destinée à être utilisée selon la revendication 5, où ladite vésicule est obtenue par un procédé incluant a) la fourniture d'une population de cellules progénitrices fibro-adipogènes (FAP), de préférence isolées à partir d'un muscle dystrophique, b) l'exposition desdites cellules *in vitro* à des inhibiteurs de HDAC et c) l'isolement d'une pluralité d'exosomes à partir desdites cellules.

7. Vésicule destinée à être utilisée selon l'une quelconque des revendications 1 à 4, où lesdits progéniteurs fibro-adipogènes (FAP) sont préalablement exposés à un ou plusieurs inhibiteurs d'histone désacétylase (HDAC) par traitement préalable d'un sujet dystrophique, animal ou humain, avec ledit inhibiteur de HDAC et isolement ultérieur desdits FAP à partir du muscle dystrophique dudit sujet.

8. Vésicule destinée à être utilisée selon la revendication 7, où ladite vésicule est obtenue par un procédé incluant a) la fourniture d'une population de cellules progénitrices fibro-adipogènes (FAP) isolées à partir d'un muscle dystrophique d'un sujet, ledit sujet étant préalablement traité avec des inhibiteurs de HDAC, et b) l'isolement d'une pluralité d'exosomes à partir desdites cellules.

9. Vésicule destinée à être utilisée selon l'une quelconque des revendications 1 à 8, où ladite vésicule comprend au moins un microARN choisi dans le groupe consistant en : miR-206-3p, miR-542-5p, miR-449a-5p, miR-342-3p, miR-320-3p, miR-192-5p, miR-423-5p, miR-376a-3p, miR-145a-5p, miR-224-5p, miR-30a-5p, miR-494-3p, miR-29a-3p et miR-7b-5p.

10. Vésicule destinée à être utilisée selon la revendication 9, qui est enrichie en au moins l'un des miARN énumérés dans la revendication 9.

11. Vésicule destinée à être utilisée selon la revendication 9 où elle comprend les microARN suivants : miR-206-3p, miR-542-5p, miR-449a-5p, miR-342-3p, miR-320-3p, miR-192-5p, miR-423-5p, miR-376a-3p, miR-145a-5p, miR-224-5p, miR-30a-5p, miR-494-3p, miR-29a-3p et miR-7b-5p.

12. Composition comprenant une pluralité de vésicules selon l'une quelconque des revendications 1 à 11, destinée à être utilisée dans le traitement d'une dystrophie musculaire.

13. Vésicule destinée à être utilisée selon l'une quelconque des revendications 1 à 11 ou composition destinée à être utilisée selon la revendication 12, où ladite dystrophie musculaire est choisie dans le groupe consistant en: la dystrophie musculaire de Duchenne, la dystrophie musculaire de Becker, la dystrophie musculaire des ceintures et d'autres formes de dystrophies musculaires et/ou les troubles musculaires partageant une pathogenèse commune avec lesdites formes.

14. Vésicule destinée à être utilisée selon l'une quelconque des revendications 1 à 11 ou composition destinée à être utilisée selon la revendication 12, où ladite dystrophie musculaire est la dystrophie musculaire de Duchenne (DMD).

15. Vésicule destinée à être utilisée selon la revendication 14, où ladite vésicule est administrée à un sujet qui est dans une phase avancée de la maladie.

16. Composition pharmaceutique contenant au moins une vésicule selon l'une quelconque des revendications 1 à 11 ainsi qu'au moins un véhicule et/ou excipient pharmaceutiquement acceptable destinée à être utilisée dans le traitement d'une dystrophie musculaire, en particulier dans le traitement de la dystrophie musculaire de Duchenne.
